(19) Europäisches Patentamt

European Patent Office

Office européen des brevets

(11) **EP 1 072 271 A2**

(12) **EUROPEAN PATENT APPLICATION**

(43) Date of publication:
**31.01.2001 Bulletin 2001/05**

(51) Int. Cl.[7]: **A61K 39/00**, A61K 35/12,
A61P 37/06

(21) Application number: **00115423.6**

(22) Date of filing: **17.07.2000**

(84) Designated Contracting States:
**AT BE CH CY DE DK ES FI FR GB GR IE IT LI LU
MC NL PT SE**
Designated Extension States:
**AL LT LV MK RO SI**

(30) Priority: **16.07.1999 US 356294**

(71) Applicant:
**ENZO THERAPEUTICS, INC.
Farmingdale, New York 11735-4716 (US)**

(72) Inventors:
• **Rabbani, Elazar
  New York, NY 10003 (US)**
• **Ilan, Yaron
  96400 Jerusalem (IL)**
• **Roy-Chowdhury, Jayanta
  New Rochelle, NY 10804-3435 (US)**
• **Engelhardt, Dean L.
  New York, NY 10024-1616 (US)**

(74) Representative:
**VOSSIUS & PARTNER
Siebertstrasse 4
81675 München (DE)**

(54) **Selective immune down regulation (SIDR) mediated transplantation processes**

(57) This invention provides for unique immune modulation applications in transplantation processes, as well as in disease intervention and prevention directed to graft-versus-host rejection as well eliminating undesirable immunological effects resulting from immunization and vaccination. Also provided are treatments for other diseases, including Crohn's disease, primary schlerosing cholangitis, primary biliary cirrhosis, atheroschlerosis, etc. This invention also provides unique compositions for carrying out such processes.

EP 1 072 271 A2

Printed by Xerox (UK) Business Services
2.16.7 (HRS)/3.6

## Description

### REFERENCE TO RELATED PATENT APPLICATIONS

This application is a continuation-in-part of U.S. Patent Application Serial No. 08/808,629, filed on February 28, 1997, and PCT International Publication No. WO98/37917, published on September 3, 1998, the contents of both applications being fully incorporated by reference herein.

### FIELD OF THE INVENTION

This invention relates to the field of immunological regulation, more particularly to selective immune down regulation, and is applicable to transplantation, disease prevention and treatment, as well as compositions used in same.

All patents, patent applications, patent publications, scientific articles, and the like, cited or identified in this application are hereby incorporated by reference in their entirety in order to describe more fully the state of the art to which the present invention pertains.

### BACKGROUND OF THE INVENTION

Although it is well known that immunization procedures have had remarkable success in halting the progression of disease spread and in the case of smallpox, eradicating it completely, the immunization process itself is not without its problems. There is a growing concern about adverse reactions to immunization procedures that can induce illness in otherwise well individuals. It is in fact the success itself of these procedures which has focused attention on the side effects of immunization. Widespread immunization programs have vastly reduced the likelihood of being exposed to a number of pathogens that were previously a major health concern, such that risk factors for developing the disease are presently overshadowed by risk factors generated by preventing the disease. As such there is a large segment of the populace that has declined immunization procedures. This in itself can allow an eruption of these previously controlled diseases. Therefore there is a need for a procedure that can allow the beneficial effects of the immunization process while ameliorating the potential deleterious effects of the immunization process.

The present invention addresses the problems and concerns discussed above by providing preventive and curative means with regard to undesirable immunological reactions and conditions, including those associated with immunization and vaccination, transplantation, and numerous immunological diseases and disorders. This invention also provides unique compositions, reagents, drugs and processes for immune modulation.

### SUMMARY OF THE INVENTION

The present invention provides a transplantation process comprising the step of establishing selective immune down regulation in a recipient, the recipient comprising immune cells, immune tissues, immune organs or components thereof from a donor, wherein SIDR is established to native or non-native antigen or antigens of the recipient or immunologically equivalent thereof, or to native or to non-native antigen or antigens of said donor in said recipient or immunologically equivalent thereof, or to both. Also provided by the present invention is a process of preventing or treating graft-versus-host rejection in a recipient using the transplantation process just described.

The present invention further provides a process for treating or preventing undesirable immunological consequences of vaccination or immunization in a subject comprising the step of establishing selective immune down regulation in the subject to any or all antigens used as a vaccine, immunization agent, adjuvant or immunologically equivalent compound, and any combination of any of the foregoing.

This invention also provides a composition of matter comprising trained or programmed cells, tissues or organ, which when introduced into a recipient exhibits or establishes selective immune down regulation in said recipient.

Also provided by this invention is a process of treating Crohn's disease in a subject comprising the steps of establishing selective immune down regulation in the subject to native or non-native antigens of the diseased cell, tissue or organ in the subject, the regulation being established by administering native or non-native antigen or antigens comprising material derived from cells, tissues, organs, or components thereof afflicted or a normal target of Crohn's disease, the native or non-native antigen or antigens being derived from allogeneic sources, xenogeneic sources, autologous sources, and combinations of such sources, and immunologically equivalent compounds and combinations of any of the foregoing.

## BRIEF DESCRIPTION OF THE FIGURES

FIGURE 1 shows reduction in MLR response in transplant donors via induction of oral tolerance towards the recipient's splenocytes.

FIGURE 2 show that induction of oral tolerance in BM donors towards recipient lymphocytes decreased skin collagen production. Collagen $\alpha 1$ (I) gene expression in skin biopsies, performed 62 days following transplantation, and hybridized with digoxigenein-labeled mice collagen $\alpha 1$ (I) probe, is shown.

FIGURE 3 shows the effect of donor tolerization on histologic evaluation of the small bowel in transplant recipients, 62 days following splenocyte transplantation.

FIGURE 4 shows oral tolerance induction in splenocytes donors towards recipients lymphocytes induce a reversal of the cytokine profile, from a Th1 to a Th2 type of response.

FIGURE 5 shows the effect of tolerance induction towards colitis and normal colon-extracted proteins on body weights in mice with experimental colitis.

FIGURE 6 shows the effect of tolerization on macroscopic grading of colitis.

FIGURE 7 shows the effect of tolerization on histologic evaluation of bowel mucosa in experimental colitis.

FIGURE 8 shows the effect of tolerization on the microscopic grading of experimental colitis.

FIGURE 9 shows the effect of oral tolerization on serum IL4 (black bars) and IFN$\gamma$ (open bars) serum levels: Tolerized mice manifested a shift form Th1 to Th2 immune response cytokine secretion.

FIGURE 10 shows that peripheral immune tolerance induction markedly enhances NK1.1 + LAL cytotoxicity functions.

FIGURE 11 shows anti-HBsAg antibody serum levels 30 days following i.p. inoculation with three different doses of the recombinant BioHepB vaccine.

FIGURE 12 shows the effect of oral administration of HBV proteins on tolerance induction towards viral antigens.

FIGURE 13 shows the effect of oral tolerance induction towards hepatitis B virus in mice with preexisting immunity towards HBV.

FIGURE 14 shows the effect of oral tolerance induction on preexisting secondary anti-HBV immune response.

FIGURE 15 shows the time course of ALT levels in *Tupaia* after HBV inoculation.

FIGURE 16 are photomicrographs illustrating liver histomorphology of normal and HBV-infected *Tupaia*.

FIGURE 17 are photomicrographs taken of *Tupaia* liver sections after *in situ* PCR amplification and detection of HBV RNA

FIGURE 18 is a time course that shows the effects of post-infection oral tolerization on the levels of antibodies to HBV antigens.

FIGURE 19 illustrates the effects of oral tolerization on the levels of antibodies to HBV antigens after HBV rechallenge.

FIGURE 20 shows the effects of pre-infection induction of oral tolerization on the levels of antibodies to HBV antigens.

FIGURE 21 shows the effects of pre-infection induction of oral tolerization on HBV induced liver damage as determined by serum ALT levels.

FIGURE 22 shows the effects of oral tolerization on HBV induced liver damage after HBV rechallenge.

FIGURE 23 are photomicrographs of *Tupaia* liver biopsy specimens that have been stained for reticulin.

## DETAILED DESCRIPTION OF THE INVENTION

The present invention provides preventive and curative means with regard to undesirable immunological reactions and conditions, including those associated with immunization and vaccination, transplantation, and numerous immunological diseases and disorders. This invention also provides unique compositions, reagents, drugs and processes for immune modulation. For prevention, the present invention allows the induction of oral tolerance to any of the components of an immunization procedure that can induce an inappropriate response. These can include but are not limited to adjuvants and compounds that are included with the antigen. For curative processes, the evocation of an inappropriate response from an immunization procedure can be treated by SIDR, GIS or combinations thereof. SIDR can be achieved by induction of oral tolerization to the components that are present with the desired antigen components in an immunization reagent or it can also include or consist of the desired antigen itself or the immunological equivalents thereof. In the former case, there will be reduction of an immune response to components other than the desired antigen while still allowing the induction of an immune response to the desired antigen. In the latter case there will be a general abrogation or reduction of the immunization process itself. GIS can be achieved either by physical processes or administration of reagents. An example of a physical process that could be used to induce GIS would be radiation treatment. Reagents that would be useful in induction of GIS could be immunosuppressive agents. Examples of immunosuppressive agents could be chemicals such as corticosteroids, cyclosporin A, FK 506 or rifampin. Examples of immunosuppressive agents could also be proteinaceous reagents such as cytokines, lymphokines, interferons, interleukins, ligands, ligand receptors, and antibodies to any of the foregoing as well as antibodies to any cells involved or associated with secondary disease manifestations including T cells.

Although down regulation of the immune system has been used to reduce the symptoms of a number of human diseases, there are other diseases that prior to the present invention have lacked recognition as being suitable candidates. In one aspect of the present invention, SIDR, GIS or any combination thereof is applied to relieve autoimmune manifestations of the following diseases: Primary sclerosing cholangitis, Primary biliary cirrhosis, Ciliac's disease, Auto-immune chronic active hepatitis, chronic liver rejection, Immune mediated liver fibrosis, Immune mediated vascular disorders, Immune mediated vascular obstruction, Immune mediated muscle disorders including smooth and striated muscles as well as blood vessel muscles.

The present invention has disclosed the relevance and treatment of undesirable immune consequences of infection by pathogens. A further example of use of the present invention is the application to treatment of coronary artery diseases in humans that may be mediated or associated with the presence of pathogens. See Mehta, J.L. et al., "Interactive role of infection, inflammation and traditoinal risk factors in atherosclerosis and coronary artery disease," Journal of Am. Coll. Cardiol. 5(6):1217-25 (l998); and Ossei-Gerning, N. et al., "Helicobacter pylori infection is related to atheroma in patients undergoing coronary angiography," Cardiovasc. Res. 35(1):120-124 (l997).

To further elaborate, it has now been demonstrated that oral tolerization leads to the production of a class of cells present at least in the peripheral blood mononuclear cell fraction (PBMC) that induce tolerization to the same antigen when adaptively transferred to syngeneic laboratory animals in this application. This identifies a class of cell that we call Ts cells. Such a population of cells is produced within an immune competent animal, including within human beings, upon the induction of tolerization including those tolerizations achieved using oral presentation of the tolerizing antigen.

In general, the present invention further demonstrates that by transferring immune cells to a recipient from a donor having previously been programmed or trained to have tolerized immune cells, one can confer SIDR in a recipient to said specific antigen or antigens by transferring these cells to a recipient (DIDR), the latter having been described in Serial No. 08/808,629, filed on February 28, 1997. The cell(s), tissue or organ (immune component) that upon transferal to the recipient will confer SIDR can be obtained from a xenogenic, allogenic, syngenic or autologous host or donor. In the instance when donor is not immunologically compatible with the immune component of the recipient and needs to carry or to be host to immune components of the recipient (surrogate doneor), it is desirable that the surrogate donor be immune deficient, immune suppressed, or tolerized to the receeipient's immune component preferably before introduction of recipient immune component to said surrogate donor such that in order to minimize the immune response of the surrogate donor to the recipient immune component. The immune component of the recipient in surrogate donor will be trained or programmed to develop a SIDR to a specific antigen or antigens (for example, by oral tolerization) which later can be transferred to the recipient and thus conferring SIDR in said recipient.

The immune cell(s), tissues or organs (immune component) that are capable of conferring SIDR in the immune recipient may be derived from PBMC, hematopoietic stem cells, liver cells, T cells, some subclass of I cell, bone marrow cells, intestinal cells, thymus cells, spleen cells, cells from lymph nodes or any combination of the above cells. Such cell(s), tissue, or organs can be derived from either a donor or a surrogate donor already having an established SIDR to a specific antigen or antigens. An alternative approach to obtaining SIDR programmed cell(s), tissues or organs is to develop, propagate and/or isolate *in vitro* or *ex vivo* such programmed immune cell(s), tissues or organs (immune components). In an example of such a process, the immune components of an already tolerized donor or surrogate donor are removed and introduced into a culture system. The specific tolerizing components may be isolated, fractionated, selected and/or are enriched for specific tolerizing activity such that only those fractions with specific tolerizing components are selected for introduction into the recipient. In this fashion, the tolerizing immune components' reaction or activity to any unwanted epitope may be avoided, if desirable, before introduction to the recipient.

Immune components are obtained or derived from an untolerized recipient and then further are subjected to an *in vitro* or *ex vivo* system through which the immune components are further trained or programmed to confer in the recipient SIDR to a specific antigen or antigens. These components may be further propagated or selectively isolated or enriched before introduction back into the recipient.

The source of immune responsive cells, tissue or organ in such a system can be an antibody producing cell, cancer cell or cell line or immune cell having the specificity for such antigen or antigens. The aforementioned antibody producing cell can be derived from a single source to provide a monoclonal antibody, or it can be derived from several sources to provide polyclonal antibodies.

The *in vitro* or culture system could be comprised of a synthetic characterized medium or a complex medium comprising noncharacterized natural components such as autologous or nonautologous serum. It further might comprise cells or tissues or organs or its fragment which may facilitate the development of a tolerizing immune component in such system and/or support propagation of such immune component. These could be derived from liver, intestine, thymus, spleen, bone marrow, or other matrices. Such an *in vitro* system could further comprise appropriate lymphokine and cytokine as they may require.

It is possible that oral tolerization induces the production of a transferring class of T cells called T suppressor cells (Ts), or other subtypes of T cells or other subtypes of lymphocytes including NK1.1 T cells. In the instance of syngeneic laboratory mice, the present invention describes the transfer of tolerance from mouse to mouse by transferring peripheral blood mononuclear cells between mice. The implication of this datum is that a specific class of cells can be produced from precursor T cells (naive T cells) in the presence of the appropriate cells, substrates and cellular factors and that it is this class of cells that confers tolerance to a specific antigen. Since the liver has been suggested as having a role in the induction of oral tolerization, subtypes of liver associated lymphocytes such as N K1.1 T cells or of other subtypes of intra-hepatc cells in the tolerizing process. It also suggests that subtypes of immune cells may be involved in programming in the liver environment via direct or indirect connection to other subtypes of immunmodulatory or other cells. Thus, if these cells could be treated outside of the body of the subject sought to be tolerized they could then be infused (perhaps after purification) back into the subject's a body to induce tolerance with a minimum of adverse effects. This "training" of a specific or even a more general subpopulation. of Ts cells could be done *in vitro* in culture or it could be done in the body of a suitable host organism. To train specific or more general Ts cells it will be necessary to establish an *in vitro* environment comparable to that which exists *in vivo* for the selective induction and expansion of antigen-specific tolerizing cells. Training may involve specific subtypes of cells and /or cytokines that will lead to reduction of an immune cell in an antigen specific or not specific manner. When trained *in vitro* for example they can be trained in a mixed lymphocyte reaction in the presence or absence of stromal cells from lymph nodes or from bone marrow or from thymus glands the liver or combinations of these three sources or other sources. When these Ts cells are trained or produced in animals they can be trained in a prepared SCID mouse or other animal (possibly with human liver cells since they have been shown to be essential to the induction of tolerization in mice). They can be trained in an animal that has been immuno ablated to sustain cell function but not cell growth. The animal can be a rodent or any animal that has a SCID condition, either genetic or induced and that can accept human liver cells if that is necessary. For example the training can be performed in a RAG2 mouse or in a rat that has been whole-body irradiated, to mention two (but not to be limited by this list.). After they have been trained and possibly amplified either *in vitro* or *in vivo*, the cells can be infused back autologously, or into an allogenic or xenogenic host or into a recipient. Either of these procedures (training or reeducation of specific or general Ts cells *in vitro* or *in vivo*) will lead to the development of noninvasive and nontoxic protocols that can manage a large number of conditions of autoimmune nature. These cells can be used as a therapeutic reagent to manage immune-mediated pathogenesis. This procedure is suitable for management of autoimmune conditions and other conditions where it is sought to minimize the effect of an immune response including humoral, including allergic response and cellular in nature.

For a further description of materials and methods and protocols used to develop the novel products and procedures of the present invention, reference is made to Chapters 1-24 of Stites, D.P. and Terr, A.I. (Eds.), <u>Basic and Clinical Immunology, 7<u>th</u> Edition</u>, Appleton & Lange, A Div. of Prentice Hall, New Jersey (l991).

For example, the appropriate calls, tissues or organs can be infused or implanted into a mouse or other animal with a severe combined immune deficiency (Rag2 mice or NOD/SCID mice, or etc.) and then the surrogate animal is tolerized through oral presentation of the tolerizing antigen or through a regiment equivalent to oral tolerization as described above. Tolerization can be accomplished by feeding the mouse 50 ng of the tolerizing antigen in 20% (v/v) of fetal bovine serum. After the completion of the tolerizing regimen these cells can be removed by leukaphoresis and if necessary isolated from the surrogate animal using conventional separation procedures such as Ficol gradients. For example if human PBMC are sought to be altered to contain functional specific Ts cells within a SCID mouse then they can be isolated from circulation and separated using a human cell-specific antibody panning procedure or some modification of such a procedure. These tolerizing cells can be infused back into the human subject to induce SDIR or a selective immune modulation.

As a further example, a Rag2 mouse containing a human-mouse chimeric liver as described in Brown et al.'s co-pending patent application, U.S. Patent Application Serial No. 08/876,635 filed on June 16, 1997, can be infused with PBMCs (or even CD45 + cells) from the person to be tolerized. The mouse can be feed with HCV antigen to induce the replication of Ts cells. Human T cells can be removed, expanded and infused back into the subject as described above. To test for the presence of specific Ts cells the mouse can be challenged with HCV subsequent to the tolerization. Observation of the absence of human cell pathology in the presence of evidence of the growth of HCV in the human cells within the liver would be evidence of the induction of tolerization.

As an additional example, an animal such as a rat can be ablated by either whole body irradiation or by using an immunoablative chemical, under conditions where the preponderance of the hematopoietic cells will not replicate, but will function according to their differentiated phenotype. PBMCs can be infused or introduced into these ablated animals and the animal tolerized through subsequent oral presentation of the tolerizing antigen or through a regimen equivalent to oral tolerization as described above. Again after the completion of the tolerization regimen these cells can be removed from and if necessary purified away from the ablated experimental animal and the immune cells from this animal.

Yet another example consists of An *in vitro* environment comparable to that which exists *in vivo* for the selective induction and expansion of antigen-specific tolerizing cells will be created. Ts cells will be produced *in vitro* or outside of the bodies of the donor or the recipient. These cells will be trained in a mixed lymphocyte reaction in the presence of antigen presenting cells as well as in the presence or absence of stromal cells from lymph nodes or from bone marrow or from thymus glands or from the liver or combinations of these three sources or other sources.

The presence or absence of Ts cells will be determined using several assay methods, including the following:

### Assay 1: $^{51}$Cr-release cytotoxicity assay:

The target cells used in these studies can be YAC-1 cells, a lymphoma cell line adapted to continuous growth in tissue culture by employing supplemented RPMI with 10% FCS, or any other type of professional target cells, or antigen specific-sensitized B or T lymphocytes. YAC-1 cells can be prepared by seeding them at a density of 2 x IO$^5$ cells/ml in 25 ml flasks with RPMI 10% FCS, and collecting them 24 hours later. Cells are suspended and collected in a 50 ml tube and washed twice with medium at 1250 rpm for 10 minutes. This procedure insures efficient labeling with $^{51}$Cr and high sensitivity of lysis by effector cells. Target cells are labeled with $^{51}$Cr, and incubated for 90 minutes at 37°C (2OOmCi/2 xIO$^6$ cells in 300µl RPMI medium). Cells are manually mixed every 10 minutes. Following incubation, 3ml of 20% FCS RPMI is added, and reincubated for 30 minutes at 37°C. Cells are washed three times in RPMI 10% FCS and counted. For determination of degree of labeling efficiency, 100 µl of cells are counted. Effector cells will be a subpopulation of lymphocytes to be tested. The $^{51}$Cr-release assay can be performed in Costar 96-well plates. A graded number of effector cells in 100µl is mixed with 5,000 labeled target cells in 100µl, with effector to target ratios (E:T ratio) of 50:1; 25:1; and 12.5:1. Each well contains target and effector cells in a total volume of 200µl. Five wells can be tested for each ratio from each sample. For determination of spontaneous release, 6 wells of a similar number of target cells are plated with 100µl RPMI 10% FCS. For determination of maximum release, 6 wells of target cells in 100µl medium are mixed with 100µl of TritonX. The plate is centrifuged for 2 minutes at 500 rpm followed by 4 hours of incubation in 5% $CO_2$ at 37°C. The plate is then centrifuged again for 2 minutes at 500 rpm and supernatants are harvested and counted using a gamma counter. Results are expressed as percent specific lysis of target cells calculated by using the equation: % cytotoxicity = mean cpm of assay-cpm from spontaneous release/cpm from targets lysed with TritonX-cpm from spontaneous release x IOO .

### Assay 2: Antigen specific cytokines release cytotoxicity assay:

Antigen specific sensitized B or T lymphocytes from serum or organ specific T cells can serve as target cells. Antigen specific tolerized B or T lymphocytes from serum or organ specific T cells can serve as effector cells. These 2 sub populations of T lymphocytes are incubated with professional antigen-presenting-cells (dendritic cells or macrophages) and with the target antigen. A cytokine release of a Thl (e.g. IFNy) or a Th2 (e.g. IL4, IL10) is measured in the supernatant by RT-PCR or by ELISA tests. T cell induction via cytokines can be used to augment the cytokine release.

### Assay 3: Measurement of reduction of amount of specific antibody produced by immune cells as a measure of presence of tolerizing activity:

These cells can be used as a therapeutic reagent to manage immune-mediated pathogenesis. These antigen-specific Ts cells can be used for reverse adoptive ransfer of tolerance. These procedures can lead to the development of noninvasive and nontoxic protocols to manage a large number of conditions of autoimmune nature.

These procedures can create a new therapy modality to manage various autoimmune-based diseases or conditions, including Hepatitis B and C infection, Crohn's disease and graft versus host disease, to name a few. The pharmaceutical material developed from this collaboration can be manufactured as an oral medication. In the case of the immune programming of a tolerizing cell this material could be delivered as an infusion of autologous cells comprising specific Ts cells or even allogenic or xenogenic cefls comprising specific Ts cells.

This class of cells could be produced outside of the body of the subject sought to be tolerized and infused (perhaps after purification) back into the subject's body to induce tolerance with a minimum of adverse effects. This "training" of a specific or even a more general subpopulation of Ts cells could be performed both *in vivo* in surrogate animals or *in vitro* in culture. When these Ts cells are trained or produced in animals they can be trained in an appropriate SCID's mouse or other animals (possibly with human liver cells that have been shown to be essential to the induction of tolerization in mice). They can be trained in an animal that has been immunoablated, and whose cells sustain cell function but will not divide. The surrogate host animal can be a rodent, or any animal that has SCID's condition either genetic or induced and that can accept human liver cells if that is necessary. For example the training can be performed in a RAG2 mouse or in a rat that has been whole-body irradiated, to mention two (but not to be limited by this list.) After specific Ts cells have been trained and possibly amplified either *in vitro* or *in vivo*, the cells can be infused back autologously, or into an allogenic or xenogenic host or recipient. To train specific or more general Ts cells in an *in vitro* environment comparable to that which exists *in vivo* for the selective induction and expansion of antigen-specific tolerizing cells (Ts) the cells, tissues or organs comprising the cells to be trained will have to be mixed with antigen presenting cells under conditions conducive of the production of Ts cells. When trained *in vitro* for example they can be trained in a mixed lymphocyte reaction in the presence or absence of stromal cells from lymph nodes or from bone marrow or from thymus glands the liver or combinations of these three sources or other sources.

Either of these procedures (training or reeducation of specific or general Ts cells *in vivo* or *in vitro*) will lead to the development of noninvasive and nontoxic protocols to manage a large number of conditions of autoimmune nature. These cells will be used as a therapeutic reagent to manage immune-mediated pathogenesis. This procedure is suitable for management of autoimmune conditions and other conditions where it is sought to minimize the undesirable effect of an immune response including humoral, including allergic response and cellular in nature.

Other embodiments and aspects of the present invention include the following directed to transplantation.

A transplantation process for preventing or reducing immunological rejection in a recipient subject comprising the steps of transplanting into a recipient subject the cells, tissues, organs, or components thereof; and establishing selective down immune regulation in the recipient transplant subject to one or more native or non-native antigens or a mixture of native and non-native antigens. Such native or non-native antigens may comprise hematopoietic cells. Such hematopoietic cells are selected from the group consisting of stem cells, T cells, lymphocytes, bone marrow cells, CD34+ and CD45+, or combinations of any of the foregoing. The aforementioned T cells may comprise NK cells or cells that express NK.1 antigens.

The native or non-native antigens can be derived from said recipient transplant subject or a donor, or both. The non-native antigens can be xenogeneic or allogeneic. The native antigens can comprise a member selected from the recipient transplant subject's stem cells, T cells and tissues expressing recipient antigens, or a combination of any of the foregoing. With respect to the non-native antigens, these may comprise stem cells or T cells from a donor.

In the transplantation process, the transplanting step can comprise bone marrow transplantation. The establishment of SIDR in the recipient transplant subject can prevent or reduce graft-versus-host (GVH) rejection which may be the result of bone marrow transplantation or solid organ transplantation.

The recipient transplant subject or a donor from whom the cells, tissues or organs, or components thereof, will be transplanted, or both, may be further subjected to one or more immune modulating treatments. Such immune modulating treatment can comprise ablation which may be carried out by a means selected from the group consisting of radiation, chemical agents, and biological agents, or a combination of any of the foregoing. The SIDR establishing step can be carried out before, during or after said ablation.

Biological agents described above are selected from the group consisting of cytokines and antibodies, or both. The immune modulating treatment may be carried out as a single dosage or repeated single dosages.

The native or non-native antigens can be selected from the group consisting of natural antigens, synthetic antigens, modified antigens, unmodified antigens, whole antigens and antigen fragments, or combinations thereof. The group of native or non-native antigens can comprise histocompatibility determinants. Such histocompatibility determinants can, in turn, comprise MHC determinants I or II, or both. The native or non-native antigens may also be selected from the group consisting of proteins, glycoproteins, enzymes, antibodies, histocompatibility determinants, ligands, receptors, hormones, cytokines, cell membranes, cell components, viruses, viral components, viral vectors, non-viral vectors, whole cells, tissues and organs. The histocompatibility determinants are MHC determinants I or II, or both, as described earlier.

Also not to be overlooked is the use of the present invention in a process for preventing or reducing undesirable immunological effect to an infectious agent in a subject, comprising establishing selective down immune regulation in the subject to either a component or components, or fragments of the infectious agent, or a native antigen, or both. The undesirable immunological effect can be selected from the group consisting of production of undesirable antibody, cell mediated immune reaction, autoimmunity, inflammatory reaction, ligand receptor binding, cytokine production and apoptosis, or a combination of any of the foregoing. The undesirable immunological effect can be caused by a member selected from the group consisting of Hepatitis and HIV. In addition, the undesirable immunological effect can be selected from the group consisting of HCV- and HBV- associated cryoglobulinemia, HCV-, HBV- and HIV-associated autoimmune disorder, HBV- and HCV-associated non-liver target organ damage.

The above-descsribed component or components or fragments can be natural, synthetic or immunologically equivalent. The transplantation process described above can further comprise the step of treating the subject with any member selected from the group consisting of a cytokine, an antibody to a cytokine, an apoptotic agent, an anti-infectious agent, or a combination of any of the foregoing.

In the just described transplantation process, the subject recipient can exhibit a bystander effect as a consequence of SIDR having been established to one or more specific antigens. Such a bystander effect may be desirable or undesirable, as the case may be.

The examples that follow are given to illustrate various aspects of the present invention. Their inclusion by no means is intended to limit in any way the scope of this invention as more particularly defined by the claims.

## DESCRIPTION OF THE PREFERRED EMBODIMENTS

The following examples are demonstrations of various aspect of the present invention as well as indicia of its value.

### Example 1 Effect of Induction of oral tolerance on GvH disease

Oral tolerization of donors with recipient antigens or immunological equivalents can abrogate a chronic Graft versus Host Disease (cGVHD) response from the donor immune cells when they are implanted into the recipient.

### MATERIALS AND METHODS

**Animals:** Donor mice were 12 weeks old B10.D2 female mice (H-2$^d$, mls-b) obtained from Jackson Laboratories (Bar Harbor, ME). Recipients were BALB/c mice (also H-2$^d$, mls-b). Mice were kept in 12 hr light/dark cycles in the Animal Core of the Hadassah-Hebrew University Medical School. All animal experiments were approved by the Hebrew-University-Hadassah Institutional Committee for Care and Use of Laboratory Animals. Following irradiation and splenocyte transplantation, mice were maintained in laminar flow isolators. All animals were fed regular laboratory chow and drank water *ad libitum*.

**Preparation and administration of recipient splenocytes to donors:** Spleens were excised from BALB/c and B10.D2 mice, and splenocytes were mechanically homogenized. After filtration through a 40-μm nylon cell strainer, remaining intact cells were spun down and removed. Proteins were measured using the Biorad Protein assay reagent. Donor B10.D2 mice were fed with homogenated splenocytes-proteins containing 50 μg of proteins, using feeding cannulae every other day (a total of 5 doses). The dose of splenocytes used was determined by initial dosing experiments performed by others as well by the inventors (Ilan at al., J Clin Invest 1997;99:109-1106, Sayegh et al., 1992;53:163-166, Sayegh et al., Immunology 1996;89: 7762-7766). The 50 μg dose was found to be in the low dose oral tolerance range.

**Splenocyte donor and recipient groups:** Two groups of mice consisting of 10 animals each were used as splenocyte donors. In the orally tolerized group (Group A'), B10.D2 donor mice were fed with homogenates of splenocytes derived from recipient strain BALB/c mice. Spleen cells from these mice were transplanted into group A BALB/c recipient mice (n = 10). The control group (Group B') consisted of donor B10.D2 mice fed with homogenates prepared from syngeneic B10.D2 mouse splenocytes, and served as spleen cell donors for group B BALB/c recipients (n = 10).

**Splenocyte transplantation**: To induce cGVHD, $2.5 \times 10^7$ spleen cells from B10.D2 mice were injected intravenously into ten BALB/c recipient mice which received $^{60}$Co whole body irradiation (6 Gy) prior to transplantation (Jaffe B.D. and Claman H.N., Cell Immunol 1985;94:73-84).

**Evaluation of tolerance induction in donor mice by one-way mixed lymphocyte reaction (MLR) assay**: Tolerance induction in donor B10.D2 mice towards minor histocompatibility antigens of recipient mice was evaluated by a one-way mixed lymphocyte reaction (MLR) test (Devender et al., In: Rose NR, Friedman H, Fahey JL, eds. Manual of clinical laboratory immunology, 3rd ed. Washington, DC: American Society for Microbiology 1986;847-852, Bishara et al., Transplantation 1994; 57:1474-1479). The one way MLR test was performed in B10.D2 splenocytes versus BALB/c splenocytes. Responding B10.D2 splenocytes ($1 \times 10^6$) were cultured in flat bottom microwell plates (Sterilin. cat. no. M29ARTL), with irradiated (30 Gy) BALB/c spleen cells ($1 \times 10^6$), in a total volume of 0.2 ml RPMI 1640 culture medium, supplemented with 100μg/ml penicillin, 100 μg/ml streptomycin, 2 mM L-glutamine, with $5 \times 10^{-5}$ 2M-ME and 10% heat-inactivated human AB serum, adsorbed into mice splenocytes. This serum combination yields a strong primary and secondary allogeneic response and a low non-specific background. Following 72 hours in a 37°C humidified 5% $CO_2$ incubator, 1mCi of $^3$H TdR (5 Ci/nmol, Nuclear Research Center, Negev, Israel) was added to each well. Cells were collected 16 to 18 hours later on paper filters, using a multiple sample harvester (Titertek Cell Harvester 530; Flow Laboratories. McClean, VA, 22102), and radioactivity was determined in a liquid scintillation counter. Background results from tolerated and non-tolerated B10.D2 splenocytes against themselves, were subtracted.

**Assessment of cGVHD**: cGVHD assessment was performed 62 days following splenocyte transplantation using the following parameters: Total body and spleen weights, collagen type $\alpha$I (I) gene expression, and liver and small bowel inflammatory response.

**Body and spleens weights**: Body weights were recorded every week throughout the study. Spleens were weighed at the end of the study.

*In situ* hybridization of collagen mRNA skin content: Mice in both groups were sacrificed 62 days following splenocyte transplantation. Skin biopsies were obtained from ears and collected in phosphate-buffered-saline (PBS). Biopsy specimens were fixed overnight in 4% paraformaldehyde in PBS at 4°C. Serial 5-μm sections were prepared after the samples had been dehydrated in graded ethanol, cleared in chloroform, and embedded in Paraplast. For hybridization, sections were deparaffinized in xylene, rehydrated in graded ethanol, rinsed in distilled water for 5 minutes, and incubated in 2 x SSC at 70°C for 30 min. Sections were again rinsed in distilled water and treated with proteinase K (10μg/ml in 50mM Tris-HCL, 5mM ethylenediaminetetraacetic acid, pH 7.5) for 10 min. After digestion, sections were blocked in 0.2% glycine, rinsed in distilled water, rapidly dehydrated in graded ethanol, air-dried for several hours, and post-fixed in 10% formalin in PBS. Skin sections were hybridized with a digoxingenin-labeled collagen α1 (I) probe generated by excising a 1600-hp insert from the plasmid (pUC18) and inserting into pSafyre (a generous gift of B.E. Kream, University of Connecticut. CT. USA), Garscon-Barre et al, J Histochem Cytochem. 1989;37:377-81, Pines et al., Matrix Biol. 1996;14:765-61, Pines at al., J Hepatol 1997;27:391-398).

**Grading of histologic changes of cGVHD**: Mice were sacrificed at 62 days following splenocyte transplantation. For evaluation of degree of hepatic and intestinal inflammation, tissue was removed from all mice in both groups and kept in 10% formaldehyde. Five tissue sections from each mouse were embedded in paraffin, sectioned and stained with hematoxylin-eosin by standard procedure. Degree of inflammation of coded microscopic sections of liver and small bowel were graded semiquantitatively from 0 to 4, as previously described, by two experienced pathologists who were unaware of the experimental conditions.

(a) Liver sections were graded according to standard scoring criteria as described by Howell et al., Bone Marrow Transplantation 1995;16: 139-145 and Howell et al., Cell Immunol 1992;140:54-66. In brief: Grade 0: normal or less than 30% portal infiltration (based on percentage of portal tracts expanded by inflammatory cells) and normal bile duct epithelium; Grade 1: 30-40% portal inflammation, and normal bile duct epithelium; Grade 2: 40-60% portal inflammation, and abnormal bile duct epithelial morphology; Grade 3: 60-80% portal inflammation and lymphocyte infiltration of bile ducts; and Grade 4: 80-100% portal inflammation, and death of bile duct cells or disruption of bile ducts.

(b) Small bowel specimens were graded by the following scale: Grade 0: normal intestinal mucosa; Grade 1: mild distortion of villous architecture with a normal amount of mucous cells, an occasional apoptotic cell in the base of crypts with evidence of cryptic hyperplasia; Grade 2: partial effacement or blunting of the villous architecture, mucous cell depletion, sloughing of epithelial cells within the lumen, marked apoptosis and crypt hyperplasia and mononuclear cell infiltration within lamina propria and cryptitis; Grade 3: as in grade 2, plus patchy or total mucosal necrosis or ulceration (Woodruff et al., Trans Proc. 1976; 8:675-684, Howell et al., 1995 op. cit).

**Serum cytokines levels**: For evaluation of the effect of oral tolerance on the balance of pro-inflammatory and anti-inflammatory cytokines, TNFα, IFNγ, IL2, and IL10, serum levels were measured by a highly sensitive RIA or enzyme immunoassay (R&D Systems, USA) according to the manufacturer's instructions, as previously described (Barak et al., 1995;17:169-173., Nagler at al., Transplantation 1995;60:943-948). These kits use an amplification system in which the alkaline phosphatase reaction product serves as a cofactor for the formation of a colored reporter system. The secondary enzyme system consists of alcohol dehydrogenase and diaphorase (amplifier). Serum cytokine levels were measured in five mice from each group 14 days after splenocyte transplantation.

**Statistical analysis**: Results were analyzed by *Student t* test for the cytokine assays, and by the Mann-Whitney Rank Sum test for the histological grading.

**RESULTS**

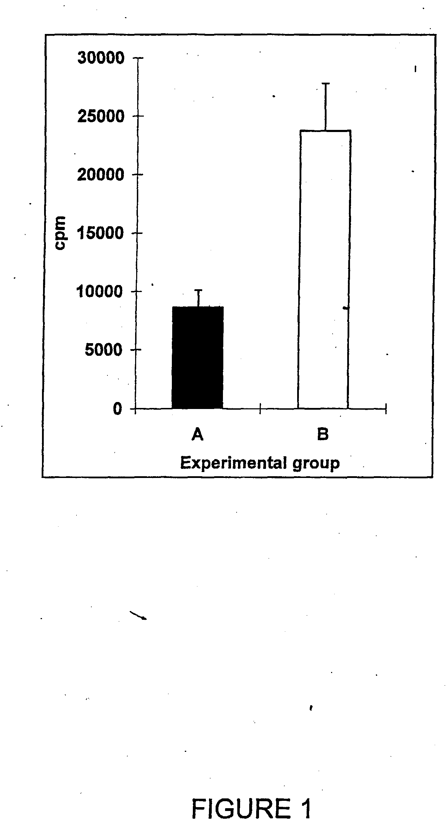

**MLR response of donor splenocytes towards recipient splenocytes**: Induction of tolerance in donor B10.D2 mice towards recipient BALB/c splenocyte antigens was measured by the one-way MLR test. As shown in FIGURE 1, responding splenocytes from tolerized B10.D2 mice manifested a marked reduction in MLR response against BALB/c target splenocytes (8648 + 1142 cpm, n = 3), as compared with responding splenocytes from non-tolerized B10.D2 mice (23,732 + 2376 cpm, n = 3, p<0.005).

**Prevention of cGVHD by oral tolerization of splenocyte donors:**

**a. Body and spleens weights**: Whole body weights 21 days following splenocyte transplantation were significantly higher in group A mice which received splenocytes from tolerized donors than in group B recipients, which received cells from non-tolerized controls (18.35 + 1.11 vs 15.68 + 0.98g, n = 10, p<0.05). However, body weights were not significantly different in either group at the end of study on day 62 (data not shown). Spleen weights were also not statistically different at the end of the study (0.058+0.0099 vs 0.456+0.014g, in recipients of tolerized and non-tolerized splenocytes respectively, n = 10).

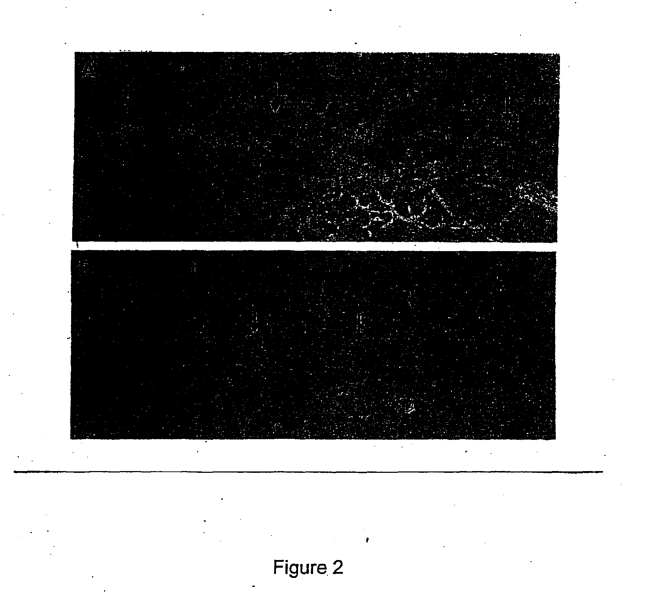

**b. Collagen 1α (I) gene expression in splenocyte recipients**: Recipients of splenocytes from donors orally tolerized to recipient splenocyte homogenates (group A) sustained less macroscopic skin thickening than did mice recipients of splenocytes from non-tolerized control donors (group B). Skin collagen α1 mRNA content was determined by in situ hybridization of skin section with a mouse collagen α1 (I) probe, 62 days following splenocyte transplantation. Mice transplanted with splenocytes from tolerized doors exhibited much less collagen α1 (I) gene expression than did mice receiving cells from non-tolerized donors (FIGURE 2, panels A and B).

**c. Prevention of cGVHD associated-liver disease by oral tolerization of the splenocyte donors**: Liver biopsies performed on recipients, 62 days following transplantation of splenocytes from non-tolerized donors (group B) showed severe portal inflammation and bile duct destruction. In contrast, recipients of splenocytes from tolerized donors (group A) showed normal liver histology in 4/10 mice. The other six mice from group A showed mild to moderate degrees of portal inflammation, lymphocyte infiltration and/or disruption of intrahepatic bile ducts. Using the standardized scoring-grading for liver and bile duct involvement in cGVHD, Group A recipients achieved a sum score of 0.2 + 0.05 compared with 2.25 + 0.5 in control group B (n = 10, p<0.005).

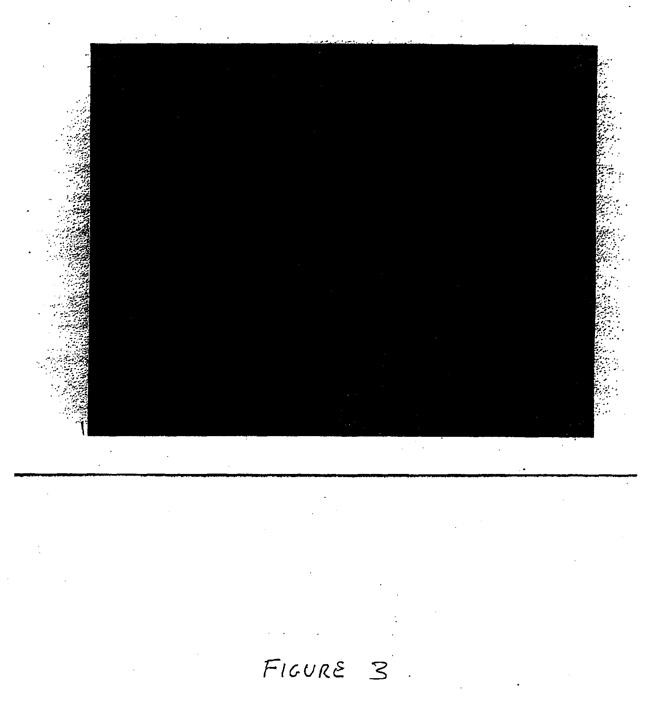

**d. Alleviation of small bowel cGVHD by oral tolerization of the donors**: Small bowel biopsies performed at the end of study in recipients of splenocytes from non-tolerized donors (group B) showed evidence of mucosal damage, distortion of villous architecture, reduction in number of mucosal cells and crypt hyperplasia, mononuclear cell infiltration within the lamina propria and cryptitis (FIGURE 3B). All features were significantly ameliorated in mice recipients of splenocytes from tolerized donors (group A, FIGURE 3A). Using the standardized score grading for small bowel involvement in cGVHD, the sum scores for small bowel involvement in group A and group B were 0.6 + 0.05 versus 2.4 + 0.8, respectively (n = 10, p<0.005).

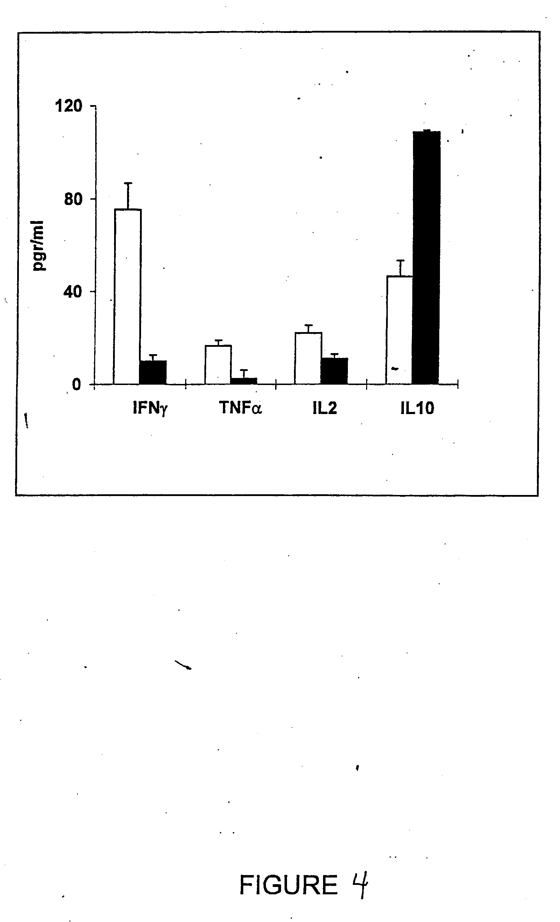

**Serum cytokine levels**: Induction of oral tolerance in splenocyte donors towards the lymphocytes of the recipient strain led to a marked change in the cytokine secretion pattern. Serum TNFα, IFNγ, and IL2 levels were decreased in mice transplanted with splenocytes taken from tolerized donors (group A), as compared with control mice from group B which received splenocytes from non-tolerized donors (2.5 + 0.88 vs.16.5 + 4.64; 10.1 + 2.6 vs 75.3 + 11.3.; and 2.1 + 0.9 vs. 22.1 + 5.55 pg/ml for TNFα, IFNγ, and IL2 respectively, p < 0.005, FIGURE 4). In contrast, serum IL-10 levels increased in mice transplanted with splenocytes from orally-tolerized donors as compared to recipients of splenocytes from group B (108.5 + 7.09 vs. 46.5 + 4.9 pg/ml, respectively, n = 5, p < 0.005, FIGURE 4).

## Summary

Oral tolerization of donors to recipient alloantigens ameliorated pathological lesions as shown by a number of different markers and symptoms associated with manifestations of cGVHD. Although the recipient alloantigens were derived from a subject different from the recipient that shared the same genotype, it should be understood that the recipient allogens could have been prepared from the recipient itself or that any immunologically equivalent antigens could have been used. Tolerization was induced by feeding homogenates of only splenocytes, yet immune attacks by transplanted lymphocytes on three major target organs of cGHVD were prevented, i.e. skin, liver and small bowel. This example demonstrates that the tolerizing reagent can provide tolerance to cells and organs that are either homologous or non-homologous to the source of the tolerizing agents.

## Example 2 Post-implantation induction of oral tolerance on GvH disease

The present example is directed towards a demonstration of oral tolerance induction in a recipient that already comprises immune cells of the donor. Oral administration of recipient antigens or immunological eqivalents can induce oral tolerization in the implanted immune cells of the donor towards the recipient. This aspect of the present invention provides improved safety and economic viability for using oral tolerization to ameliorate cGVHD. By the means shown in this example, the safety of the donor does not have to be compromised by being immune suppressed. In addition, the donor is not subject to exposure to any disease agents in the recipient that were responsible for the necessity of a transplant. The donor is not subject to exposure to disease or agents that may also be fortuitously present in the intended recipient. This aspect of the invention thereby avoids the possibility of transmission of such diseases or agents to the donor and/or induction of oral tolerance to such diseases or agents in the donor.

This example was carried out as described previously in Example 1 with the exception that oral tolerance was induced by feeding the recipient BALB/c mice with proteins extracted from BALB/c splenocytes at 50υg/day per mouse for 11 days following transplantation of B10.D2 donor splenocytes. Assessment of the effectiveness of the post-implantation tolerance induction in the donor immune cells was carried out as described in the previous example. There was significant reduction in mixed lymphocyte response of effector splenocytes from tolerized BALB/c mice transplanted with B10.D2 splenocytes against BALB/c target splenocytes (32 cpm) compared with non-tolerized controls (546 cpm). Oral tolerization ameliorated liver inflammation and bile duct destruction and the inflammatory bowel changes of cGVHD. Tolerized recipients showed less reduction in weight and subcutaneous fat, and had much less skin thickening and collagen deposit. Reduction of collagen $\alpha$ 1 (I) gene expression was shown by in situ hybridization with collagen $\alpha$ 1 (I) probe. Serum IL-10 levels were significantly higher in the tolerized mice than in controls whereas levels were significantly reduced.

## Example 3 Mutual tolerization

A further aspect of the present invention is that mutual tolerization can be carried out. In this aspect, in addition to abrogation of cGVHD by tolerizing the donor immune cells to antigens displayed by recipient cells and organs, there can be induction of oral tolerance to down regulate the reaction of the recipient immune system to the presence of the donor cells or organs. As disclosed in the present invention, this can be carried out either during or prior to an implantation procedure. Also as described in the present invention, the tolerizing agents can be derived from donors, recipients, recipients that comprise donor cells, immunological equivalents of any of the fooregoing and combination thereof. This can serve to eliminate the need for ablation, to reduce the amount of ablation or to eliminate immune reactions to donor cells that are invoked in recipient immune cells that have inadvertently evaded the ablation treatment.

## Example 4 Effect of induction of oral tolerization on experimental colitis

The present example is a further demonstration that oral tolerization can be used at the same time that colitis is being induced. This example also serves as a demonstration that colon-extracted proteins from normal donors as well as donors with colitis can be used to induce oral tolerization. The present example is also a demonstration that colon-extracted proteins from one species can be used to induce oral tolerance and alleviate colitis in a different species. The ability to alleviate experimental colitis by the induction of oral tolerization with surrogate allergens creates a viable methodology that will be of use in human therapy. For instance, a necessity for using diseased tissues as tolerant sources could increase the likelihood of a transfer of the disease inducer as well as tolerizing factors. By the disclosure in the present invention that the tolerizing proteins do not have to be derived from a donor that exhibits the disease, this and other potential problems are avoided. The nature of the species used as a source of material to be used as a tolerizing agent in humans also brings up ethical and moral issues. The ability to use different species as a source of tolerizing agents allows the donor to be xenogeneic thereby avoiding such issues. Tolerization reagents that are derived from the same species also have an intrinsic risk that there may be pathogens or epitopes of pathogens present in the reagents such that administration of the oral tolerization reagent also inducts tolerance to the presence of that pathogen. This can provoke a disease state at the time of the tolerization if there are viable pathogens in the tolerization reagents or if there are viable pathogens resident within the recipient. In the absence of viable pathogens at the time of the tolerization induction, there is still the possibility that an inadvertent tolerization to a pathogen may allow induction of a disease process when the recipient is exposed to the pathogen at a later time. The ability to use either xenogeneic or immunologically equivalent sources as tolerization reagents can either decrease or eliminate the possibility of this event. It is also understood that these considerations are equally applicable to other aspects of the present invention.

## MATERIALS AND METHODS

**Animals**: Normal inbred 2-4 months BALB/c male mice were obtained from Harlan Laboratories and maintained in the Animal Core of the Hadassah-Hebrew University Medical School. Mice were maintained on standard laboratory chow and kept in 12 hr light/dark cycles. All animal experiments were carried out according to the guidelines of the Hebrew-University-Hadassah Institutional Committee for Care and Use of Laboratory Animals and with the committee's approval.

**Induction of colitis**: TNBS-colitis was induced by rectal instillation of 2,4,6-trinitrobenzen sulfonic acid (TNBS), 1mg /mouse, dissolved in 100µl of 50% ethanol as described by Neurath et al., J Exp Med 1995;182:1281-1290).

**Preparation and administration of the oral antigen and bystander antigens:** Colon was removed from normal or from TNBS-induced-colitis-colons of mice, rats, rabbits and human specimens, cut into small strips and mechanically homogenized. After filtration through a 40-µm nylon cell strainer, the intact cells were spun down and removed. Proteins were quantified using a protein assay kit (Biorad). Normal colon extracted proteins (NCEP), and colitis extracted proteins (CEP), were orally administered into the experimental groups described below, using a feeding atraumatic needle, every other day for 10 days (a total of 6 doses). For preparation of human colon specimens, colon samples were exposed *in vitro* to TNBS as described before (Neurath at al., 1995; *supra*.).

**Experimental groups**: Nine groups of mice, consisting of 10 animals each, were studied as shown in Table 1 below.

Table 1

| Experimental and control groups | | | |
|---|---|---|---|
| Group: | Antigen fed: | Oral antigen administration days: | Day of tolerance evaluation: |
| A | CEP-mouse | 0-10 | 12 |
| B | CEP-rat | 0-10 | 12 |
| C | CEP-rabbit | 0-10 | 12 |
| D | CEP-human | 0-10 | 12 |
| E | NCEP-mouse | 0-10 | 12 |
| F | NCEP-rat | 0-10 | 12 |
| G | NCEP-rabbit | 0-10 | 12 |
| H | NCEP-human | 0-10 | 12 |
| I | BSA | 0-10 | 12 |
| NCEP: Normal colon extracted proteins  CEP: Colitis extracted proteins  BSA: Bovine serum albumin | | | |

All mice were challenged with rectal TNBS on day 1 of the study. Mice in all groups were fed (50μg/mouse) every other day for 10 days starting on day of colitis induction. Group A through D included mice fed with colitis extracted proteins (CEP). Group A mice were fed with mouse-derived-CEP; Group B were fed with rat-CEP; Group C mice were fed with rabbit-CEP; and Group D were fed with human-CEP. Groups E to H were fed with normal colon-extracted- proteins (NCEP): Group E was fed with mouse-derived NCEP; group F with rat-NCEP; group G with rabbit-NCEP; and group H with human-NCEP. Control mice in group I were fed with bovine serum albumin (BSA). Mice in all groups were sacrificed 12 days following colitis induction.

**Evaluation of the effect of surrogate antigens on tolerance induction in the experimental colitis model**: The role of surrogate antigens in tolerance induction was evaluated by monitoring the following parameters for colitis:

(a) **Clinical assessment of colitis**: Diarrhea was followed daily throughout the study.
(b) **Macroscopic score of colitis**: Colitis assessment was performed 12 days after colitis induction using standard parameters (Neurath et al., 1995 *supra*.). Four macroscopic parameters were determined, namely: degree of colonic ulcerations; intestinal and peritoneal adhesions; wall thickness; and degree of mucosal edema. Each parameter was graded on a scale from 0 (completely normal) to 4 (most severe) by two experienced blinded examiners.
(c) **Grading of histological lesions**: For histological evaluation of inflammation, distal colonic tissue (last 10 cm) was removed and fixed in 10% formaldehyde. Five paraffin sections from each mouse were then stained with hematoxylin-eosin by using standard techniques. The degree of inflammation on microscopic cross sections of the colon was graded semiquantitatively from 0 to 4 (Neurath et al., 1995, *supra*.). Grade 0: normal with no signs of inflammation; Grade 1: very low level of leukocyte infiltration; Grade 2: low level of leukocyte infiltration; and Grade 3: high level of infiltration with high vascular density, and bowel wall thickening; Grade 4: transmural infiltrates with loss of goblet cells, high vascular density, wall thickening, and disruption of normal bowel architecture. The grading was performed by two experienced-blinded pathologists.

**Evaluation of possible indicators related to induction of immune tolerance:** Serum IL4, and IFNγ levels were measured by a "sandwich" ELISA, using Genzyme Diagnostics kits (Genzyme Diagnostics, MA, USA) according to the manufacturer's instructions. Serum levels were measured in all mice from all experimental and control groups 10 days after colitis induction.

**Evaluation of the effect of tolerance induction on NK1.1 liver-associated-lymphocytes:**

(a) **Liver lymphocytes isolation**: The inferior vena cava was cut above the diaphragm and the liver was flushed with 5 ml of cold PBS until it became pale. The connective tissue and the gallbladder were removed, and livers were placed in a 10 ml dish in cold sterile PBS. Livers were crushed through a stainless mesh (size 60, Sigma Chemical Co., St. Louis, MO). Cell suspension was placed in a 50-ml tube for 3 minutes and washed twice in cold PBS (1250 rpm for 10 minutes), and debris was removed. Cells were resuspended in PBS, and the suspension was placed through a nylon mesh presoaked in PBS. Unbound cells were collected. Cells were washed twice in 45 ml PBS (1250 rpm at room temperature). For liver lymphocyte isolation, 20 ml of Histopaque 1077 (Sigma Diagnostics, St. Louis, MO) was slowly placed beneath the cells and suspended in 7 ml of PBS, in a 50-ml tube. The tube was centrifuged in 1640 rpm for 15 minutes at room temperature. Cells at the interface were collected, diluted in a 50 ml tube, and washed twice with ice-cold PBS (1250 rpm for 10 minutes). Approximately $1\times10^6$ cells/mouse liver were recovered. The viability by trypan blue staining was more than 95%.

**(b) Flow cytometry analysis for determination of NK1.1 cell depletion:** Immediately after lymphocyte isolation, duplicates of $2\text{-}5\times10^4$ cells/500µl PBS were put into Falcon 2052 tubes incubated with 4 ml of 1 % BSA for 10 minutes, and centrifuged at 1400 rpm for 5 minutes. Cells were resuspended in 10µl FCS with 1:20 FITC-anti mouse NK1.1 antibody (NKR-P1C, Pharmingen, USA), and mixed every 10 minutes for 30 minutes. Cells were washed twice in 1 % BSA, 0.5 ml 1% paraformaldehyde was added, and kept in 4°C until reading. For the control group, only 5µl of 1% BSA was added. Analytical cell sorting was performed on $1\times10^4$ cells from each group with a fluorescence-activated cell sorter (FACSTAR plus, Becton Dickinson). Only live cells were counted, and background fluorescence from non-antibody-treated lymphocytes were deducted from the levels obtained.

**(c) Liver lymphocytes cytotoxicity assays**: The target cells used in these studies were YAC-1 cells, a lymphoma cell line adapted to continuous growth in tissue culture by employing supplemented RPMI with 10% FCS. YAC-1 cells were prepared for NK assay by seeding them at a density of $2\times10^5$ cells/ml in 25 ml flasks with RPMI 10% FCS, and collecting them 24 hours later. Cells were suspended and collected in a 50 ml tube and washed twice with medium at 1250 rpm for 10 minutes. This procedure ensured efficient labeling with $^{51}$Cr and high sensitivity of lysis by NK cells. Target cells were labeled with $^{51}$Cr (New Life Science, Boston MA, Gamidor, Israel) and incubated for 90 minutes at 37°C ($200\text{mCi}/2\times10^6$ cells in 300µl RPMI medium). Cells were manually mixed every 10 minutes. Following incubation, 3ml of 20% FCS RPMI were added, and reincubated for 30 minutes at 37°C. Cells were washed three times in RPMI 10% FCS and counted. For determination of degree of labeling efficiency, 100µl of cells were counted, and a minimum of 0.6 cpm/cell was measured. Effector cells were liver lymphocytes isolated from livers from groups A-H described above. The $^{51}$Cr-release assay was performed in Costar 96-well plates. A graded number of effector cells in 100µl were mixed with 5000 labeled target cells in 100 µl, with effector to target ratios (E:T ratio) of 25:1. Each well contained target and effector cells in a total volume of 200µl. Five wells were tested for each ratio from each sample. For determination of spontaneous release, 6 wells of a similar number of target cells were plated with 100µl RPMI 10% FCS. For determination of maximum release, 6 wells of target cells in 100µl medium were mixed with 100µl TritonX. The plate was centrifuged for 2 minutes (500 rpm) followed by 4 hours of incubation in 5%$CO_2$ at 37°C. The plate was than centrifuged again for 2 minutes (500 rpm), and supernatants were harvested and counted using a gamma counter. Results were expressed as percent specific lysis of target cells calculated by using the equation: % cytotoxicity = mean cpm of assay-cpm from spontaneous release/cpm from targets lysed with TritonX-cpm from spontaneous release x100 .

**RESULTS**

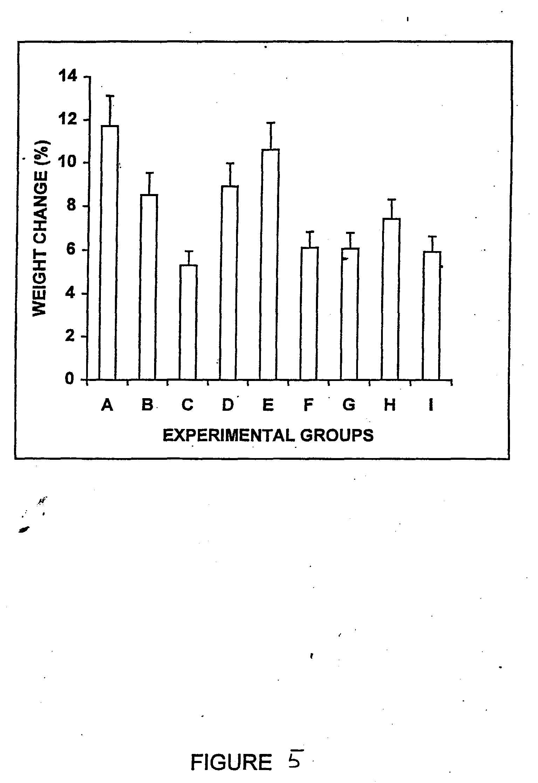

**Clinical assessment of colitis:** A marked decrease in diarrhea was observed in tolerized mice from group A fed with mouse-CEP. A similar beneficial effect was observed in mice treated with mouse derived-NCEP in group E. In contrast, mice fed with rabbit-CEP or with NCEP from rat, rabbit or human colons in groups C,F,G,H, respectively, and mice in control group I, fed with BSA, suffered severe diarrhea. Follow up of mice body weight disclosed a statistically significant increase in body weight among tolerized mice in group A compared with control mice in group I (11.7% vs. 5.9%, respectively, p<0.005, FIGURE 5). A similar beneficial effect was observed in mice from group E treated with mouse-derived NCEP, and their weight increased by 10.6% (p<0.005). A favorable effect was also observed in mice fed with rat and human CEP (8.5% and 8.9%, increase in body weights for groups B and D, respectively). In contrast, no significant increase in body weight was noted in mice treated with rabbit-CEP, or with rat, rabbit, or human-NCEP in groups C, F, G, H, and their body weights increased by 5.3, 6.1, 6.05, and 6.9% respectively (FIGURE 5).

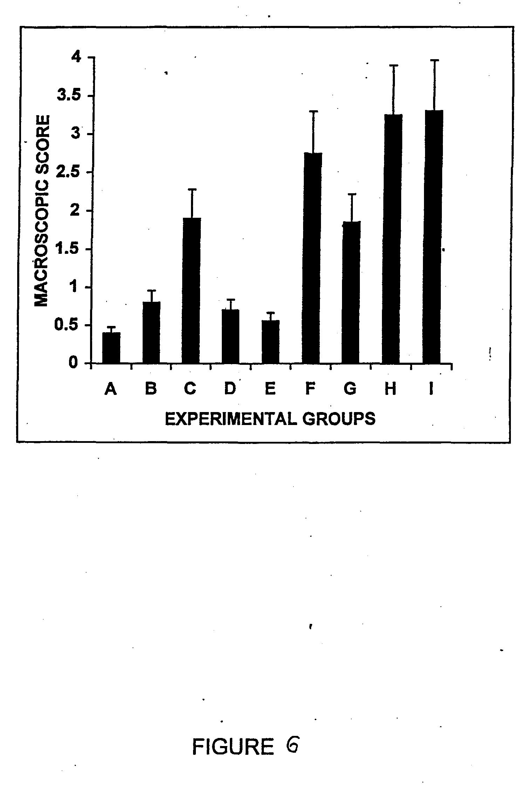

**Macroscopic grading of colitis**: Induction of oral tolerance by feeding of mouse extracted colitis-derived proteins (group A), or with mouse-normal colon extracted proteins (group E), markedly alleviated the macroscopic grading of colitis. The scores for tested macroscopic parameters of colitis were: degree of colonic ulceration, intestinal and peritoneal adhesions, wall thickness, and degree of mucosal edema. The total macroscopic score was 0.4 and 0.56 in groups A and E mice respectively, compared with 3.3 in the non-treated control group I (p<0.005, FIGURE 6). A favorable effect was also observed in mice fed with rat and human-derived CEP (groups B and D). Their total macroscopic scores measured 0.8 and 0.7, respectively. In contrast, non-tolerized mice, fed with rabbit-CEP or with NCEP derived from rat, rabbit or humans (groups C, F, G and H respectively) suffered from severe colitis, and their total macroscopic scores were 1.9, 2.75, 1.85, 3.25 respectively. The total score in non treated-control mice was 3.3 (group I, FIGURE 6).

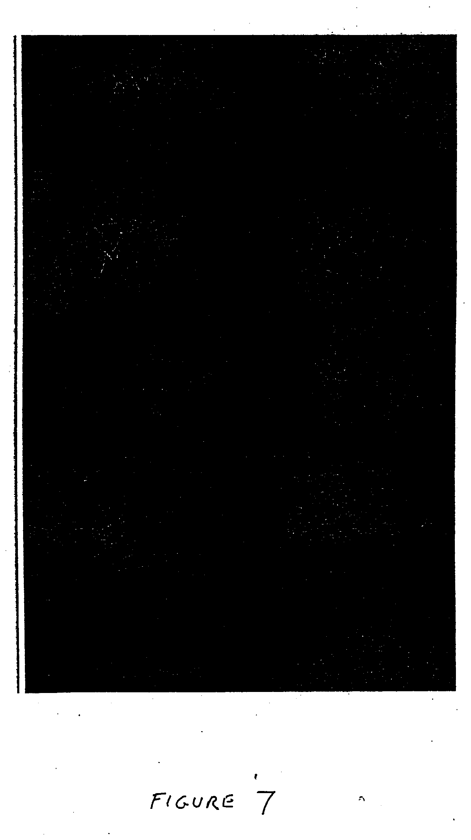

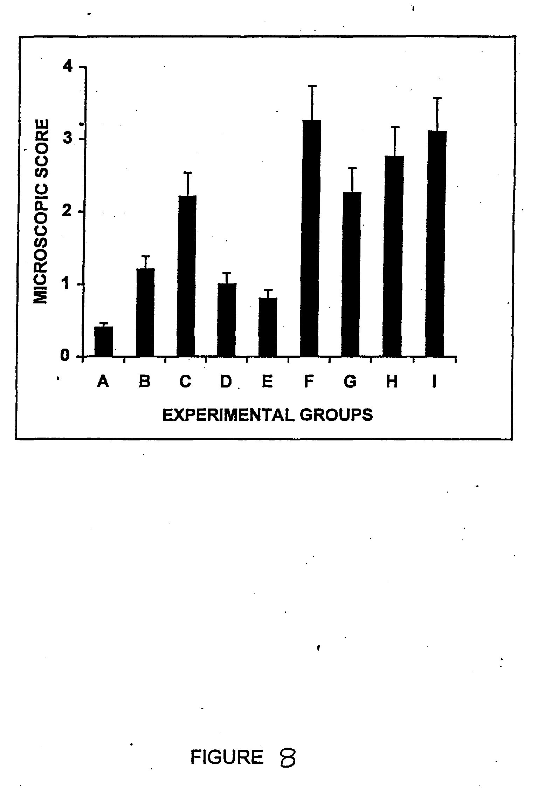

**Grading of histological lesions**: Histologic evaluation of bowel tissue showed a marked reduction in inflammatory response and mucosal ulcerations in tolerized mice in groups A and E, compared with non-tolerized mice in groups C, F, G, and H and compared with control mice from group I. In mice in groups A and E, almost normal sections, or only minimal lymphocytic infiltration was detected (FIGURE 7, panels A and E). In contrast, severe inflammatory reaction (grade 3-4) was observed in bowel specimens taken from non-tolerized mice (FIGURE 7, panel F). A partial effect was observed in mice fed with rat and human derived CEP in groups B and D (FIGURE 7, panel B). The results of standard pathological tests in tolerized mice from groups A and E produced scores of 0.4 and 0.8 versus 2.2, 3.25, 2.25, and 2.75 for non-tolerized mice from groups C, F, G, and H, respectively (FIGURE 8). Control non-treated mice in group I exhibited severe microscopic colitis with a pathological score of 3.1 (not shown).

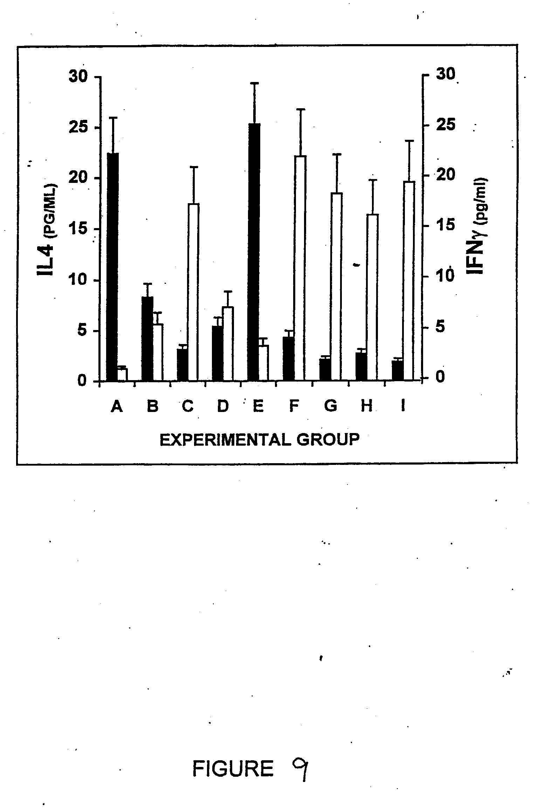

**Serum IL4, and IFNγ levels:** Tolerized mice manifested a shift from Th1 to Th2 immune response cytokine secretion. The feeding of mouse-derived CEP induced an increase in IL4 levels and a decrease in IFNγ serum levels 10 days following colitis induction, to 22.4±5 pg/ml and 1.2±0.7 pg/ml respectively (group A, p<0.005 compared with control group I). Similarly, the feeding of mouse-derived NCEP induced a Th1 to Th2 immune shift with IL4 and IFNγ serum levels of 25.4±5.7 and 3.5±0.9 respectively (group E, p<0.005, FIGURE 9). In contrast, mice from non-tolerized groups C, F,G, H and control group I, exhibited high IFNγ and low IL4 serum levels (17.4±3.7 and 3.1±0.9; 22.1±4 and 4.3±1.1; 18.4±3.8 and 2.1±0.8 ;16.3±2.9 and 2.7±0.7; 19.5±4.2 and 1.9±0.4 respectively, FIGURE 9).

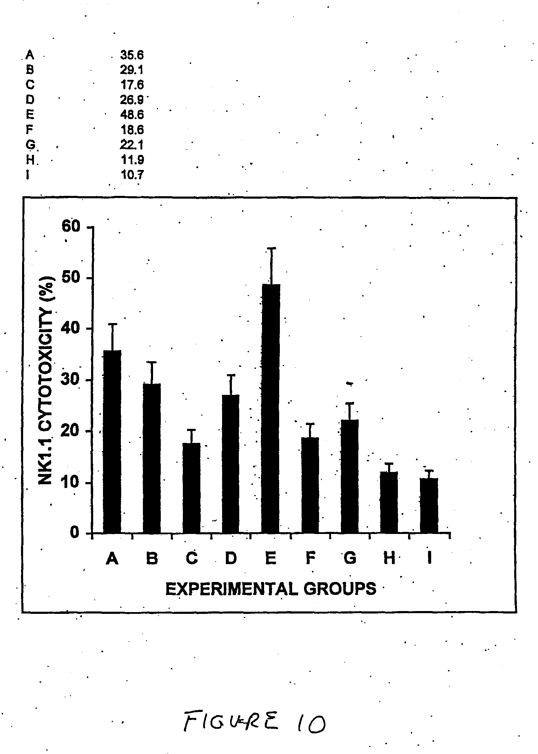

**Tolerance induction enhanced NK1.1 + LAL cytotoxicity functions:** Intrahepatic lymphocytes were isolated from all groups of treated and control mice. Analytical cell sorting was performed on cells from each group following lymphocyte isolation using FITC-anti mouse NK1.1 antibody. Induction of tolerance by feeding of mouse-derived CEP and mouse-derived NCEP markedly enhanced NK1.1 + LAL-cytotoxicity function, as measured by specific lysis assays. YAC-1 cells were used as target cells in these studies at an E:T ratio of 25:1. Liver lymphocytes isolated from tolerized mice in groups A and E showed increased cytotoxicity, compared with non tolerized-control mice in group I (35.6% and 48.6% vs 10.7% in Groups A and E vs Group I, respectively, p<0.005, FIGURE 10). A similar effect was observed in mice from groups B and D, in which cytotoxicity rates measured 29.1% and 26.9% respectively. In contrast, non-tolerized mice from groups C, F, G, and H, manifested significantly lower NK1.1 + LAL cytotoxicity-rates (17.6%, 18.6%, 22.1 %, and 11.9%, respectively, FIGURE 10). Rectal TNBS instillation had no effect on cytotoxicity assays (10.7%, group I).

### Example 5 Post-colitis induction of oral tolerance

It was further demonstrated that oral tolerization can be used to control symptoms of colitis even after the colitis has been established in a subject. This example was carried out using the same procedures described in the previous example with the exception of the following:

**Experimental groups:** Six groups of mice, consisting of 10 animals each, were studied (Table 1). All mice were challenged with rectal TNBS on day 1 of the study. Mice in groups A, B, and E, were fed every other day for 10 days starting on the day of colitis induction. For evaluation of the effect of tolerance induction on established experimental colitis, mice in groups C, D, and F were fed with similar doses starting on day 7, following induction of colitis. Mice in groups A and C were fed with colitis extracted proteins (CEP). For evaluation of the effects of surrogate antigens on tolerance induction, mice in groups B and D were fed with normal colon-extracted- proteins (NCEP). Mice in control groups E and F were fed with similar doses of bovine serum albumin (BSA). Mice in all groups were sacrificed 21 days following colitis induction.

Evaluation of the effects of oral tolerance induction was carried out as described in the previous example.

### Results

**Clinical assessment of colitis**: A marked decrease in diarrhea was observed in tolerized mice from group A fed with mouse-CEP. A similar beneficial effect was observed in mice treated with mouse derived-NCEP in group B, that were fed starting on day 1. A partial beneficial effect was observed in mice in which tolerance was induced in the presence of established colitis (groups C and D). In contrast, mice in control groups E and F fed with BSA, suffered severe diarrhea. A follow up observation of mice body weight disclosed a statistically significant increase in body weight among tolerized mice in group A compared with control mice in group E (11.7% vs. 4.2%, respectively, p<0.005). A similar beneficial effect was observed in mice from group B treated with mouse-derived NCEP, and their weight increased by 8.9% (p<0.005). A favorable effect was also observed in mice fed with CEP and NCEP starting 7 days following induction of colitis (9.3% and 7.2% increase in body weights for groups C and D, respectively). In contrast, no significant increase in body weight was noted in mice treated with BSA. Their body weights increased by 4.2 and 4.1 % respectively for mice in groups E and F.

**Macroscopic grading of colitis**: Induction of oral tolerance by feeding of mouse extracted colitis-derived proteins (group A), or with mouse-normal colon extracted proteins (group B), markedly alleviated the macroscopic grading of colitis. The scores for tested macroscopic parameters of colitis were: degree of colonic ulceration, intestinal and peritoneal adhesions, wall thickness, and degree of mucosal edema. Total macroscopic scores measured 0.4 and 0.7 in groups A and B mice respectively, compared with 3.3 and 3.1 in non-treated controls in groups E and F (p<0.005). A favorable effect was also observed in mice fed with CEP starting 7 days following colitis induction (groups C). Their total macroscopic score measured 1.2. A partial beneficial effect was noted in mice fed with NCEP starting 7 days after colitis induction, and their scores were 1.9.

**Grading of histological lesions**: Histologic evaluation of bowel tissue showed a marked reduction in inflammatory response and mucosal ulcerations in tolerized mice in groups A, B C and D, compared with non-tolerized control mice in groups E and F. In mice in groups A and B, almost normal sections, or only minimal lymphocytic infiltration was detected. In contrast, severe inflammatory reaction (grade 3-4) was observed in bowel specimens taken from non-tolerized control mice. A partial effect was observed in mice fed with CEP or NCEP starting 7 days following colitis induction in groups C and D. The results of standard pathological tests in tolerized mice from groups A, B, C, and D produced scores of 0.4, 1.2, 1.4, and 1.95, respectively. Control non-treated mice in groups E and F exhibited severe microscopic colitis, with pathological scores of 3.1 and 3.25, respectively.

**Serum IL4, and IFNγ Levels:** Tolerized mice manifested a shift from a Th1 to Th2 immune response cytokine secretion. The feeding of mouse-derived CEP induced an increase in IL4 levels end a decrease in IFNγ serum levels 10 days following colitis induction, to 22.4±5 pg/ml and 1.2±0.7 pg/ml respectively (group A, p<0.005 compared with control group E). Similarly, the feeding of mouse-derived NCEP induced a Th1 to Th2 immune shift with IL4 and IFNγ, serum levels of 18.4±1.9 and 2.4±0.9 respectively (group B, p<0.005). Primed mice that were tolerized in the presence of pre-existing disease fed with CEP or NCEP, exhibited a similar cytokine paradigm, and their IL4 levels increased to 16.4±2.9 and 10.3±1.1, while their IFNγ serum levels measured 6.3±0.9 and 7.3±1.1 (groups C and D, respectively). In contrast, mice from non-tolerized control groups E and F, exhibited high IFNγ and low IL4 serum levels (18.4±2.7 and 1.9±0.3; and 22.1±1 and 2.1±0.5; respectively).

**Example 6 Induction of oral tolerization to HBV antigens in mice**

One of the aspects of the present invention is the application of SIDR and/or GIS to abrogate the induction of secondary manifestations that are associated with infection by a pathogen. These manifestations are defined as secondary to the infection in that they are not directly related to replicative processes of the pathogen. Illustrative examples of such secondary manifestations are pathological processes such as cytotoxicity, induced auto-immunity and apoptosis. In subjects where the secondary manifestations have already been presented, the present invention would lead to prevention of further progression or reduction of disease symptoms.

In one aspect of the present invention, SIDR or GIDS or any combination thereof is used with a subject in order to relieve an undesirable immune response that is a consequence of infection by a pathogen. The present disclosure describes the utility that the present invention would have as a therapeutic approach for prevention and/or treatment of the hepatic damage associated with HBV Infection. Other examples of pathogens include HCV, CMV, retroviruses such as HIV and bacteria such as Streptomyces, Helicobacteria and Chlamydia. Infection by these pathogens has been associated with diseases that may be derived from autoimmune dysfunctions. These diseases can include Hepatitis, AIDS, atherosclerosis, atheroma, thrombosis, coronary artery disease and myocardial infarcation (Mehta et al., J Am Coll Cardiol 1998; 31:1217-1225, Gurfinkel and Bozovich, Atherosclerosis 1998;140 Suppl 1:S31-35, Ossei-Gerning et al., Cardiovasc Res 1997;35:120-124, Anderson at al., J Am Coll Cardiol 1998;32:35-41, Biasucci et al., 1999;99:855-860).

The following example is a demonstration that it is possible to induce immune tolerance towards HBV antigens and that oral tolerance can be used for down regulation of a pre-existing immune response to HBV antigens.

**MATERIALS AND METHODS**

**Animals:** Normal inbred, female BALB/c mice (25-30 gram), were obtained from the Animal Core of the Hadassah-Hebrew University Medical School. Mice were maintained on standard laboratory chow and kept in 12 hr light/dark cycles. All animal experiments were carried out according to the guidelines of the Hebrew-University-Hadassah Institutional Committee for Care and Use of Laboratory Animals, and with the committee's approval.

**Induction of anti-HBV immune response**: BioHepB recombinant hepatitis B vaccine (BioTechnology General LTD, Israel), which contains three surface antigens of the hepatitis B virus: HBsAg, PreS1 and preS2, was used for induction of anti-HBV immune response (Shouval et al., Vaccine 1994;12:1453-1459). This vaccine was chosen as it has been shown previously to improve immunogenicity compared to other vaccines, and induces a high level of seroconversion and high antibody titer (Ibid.). We tested the optimal vaccine dose required for induction of an effective anti-viral immune response. Three groups of mice consisting of 10 animals each were studied. Mice in each group were injected intra-peritoneal (i.p.) with one of three doses of the BioHepB vaccine: 0.2, 0.4, or 0.8 mcg. Mice were followed for serum anti-HBs antibody titers 30 days after vaccine inoculations.

**Induction of oral tolerance towards HBV antigens**: Recombinantly prepared HBsAg + PreS1 + PreS2 antigens (BioHepB, BioTechnology General LTD, Israel) were used as target antigens for induction of peripheral immune tolerance in the present study. Peripheral tolerance was induced through oral administration of low dose HBV antigens (BioHepB), 1mcg/feeding, using a feeding atraumatic-needle, on alternate days for 10 days (a total of 5 doses). Control groups received similar doses of bovine serum albumin (BSA).

**Experimental groups**: Eight groups of mice consisting of 10 animals each were studied as shown in Table 2 below.

TABLE 2

| Experimental and control groups: | | | | |
|---|---|---|---|---|
| Group: | Antigen fed: | Days of feeding: | Days of BioHepB vaccination | Days of anti-HBs evaluation: |
| A | None | None | 1, 30 | 30, 60 |
| B | HBsAg + pre S1 + pre S2 | 1 to 10 | 10, 40 | 40, 70 |
| C | BSA | 1 to 10 | 10, 40 | 40, 70 |
| D | HBsAg + pre S1 + pre S2 | 1 to 10 | 1, 30 | 30, 60 |
| E | BSA | 1 to 10 | 1, 30 | 30, 60 |
| F | HBsAg + pre S1 + pre S2 | 30 to 40 | 1, 30 | 30, 60 |
| G | BSA | 30 to 40 | 1, 30 | 30, 60 |
| H | HBsAg + pre S1 + pre S2 | 1 to 10 | None | 30, 60 |

Experimental groups A to G were inoculated twice i.p. with the BioHepB. vaccine (0.04mcg) at one-month intervals. Mice in control group A were vaccinated on days 1 and 30 without oral feedings, and were followed for serum anti-HBs antibody titers 30 days after each injection on days 30 and 60. To determine the effect of oral tolerance induction on the humoral anti-HBV immune response in naive animals, mice in-groups B and C were fed with HBV-envelope proteins (BioHepB) or bovine serum albumin (BSA), respectively. This was followed by two inoculations i.p. with the Bio-HepB vaccine on days 10 and 40. Mice were followed for serum anti-HBs titers thirty days following each immunization, on days 40 and 70. To determine the effect of oral tolerance induction on pre-existing anti-HBV immune response, mice in-groups D and E were inoculated i.p. with the BioHepB vaccine followed by oral administration of HBV-envelope proteins (BioHepB) to mice in experimental group D, or BSA to mice in control group E, beginning on vaccination day. Mice in both groups were re-immunized with the BioHepB vaccine on day 30, and anti-HBs antibody titers were measured 30 days following the second injection on day 60. For further determination of the effect of oral tolerance induction on anti-HBV immunity, mice in-groups F and G were inoculated twice with the BioHepB vaccine on days 1 and 30, followed by oral administration of HBV-envelope-protein (BioHepB) to mice in experimental group E, or of BSA to mice in control group G. Mice were followed for anti-HBs titers before oral tolerance induction on day 30, and following tolerance induction on day 60. To determine the effect of oral administration of HBV-envelope proteins (BioHepB) on anti-HBs titers, mice in group H received 5 oral doses of HBV antigens without vaccination. Mice were followed 30 days later for development of anti-HBs serum levels.

**Assessment of anti-HBs humoral immune response:** Antibodies to HBsAg were detected by a commercial solid phase radioimmunoassay (RIA, Ausab, Abbott Laboratories, North Chicago, IL). A World Health Organization reference serum was used for quantitative analysis of anti-HBs by RIA, utilizing the Hollinger formula and expressed in mIU/ml/ml. Mice in all experimental and control groups were followed for anti-HBs antibody titers 30 days following each inoculation of the BioHepB vaccine, throughout the study.

**Statistical analysis**: Results were analyzed using the standard student *t* test.

**RESULTS**

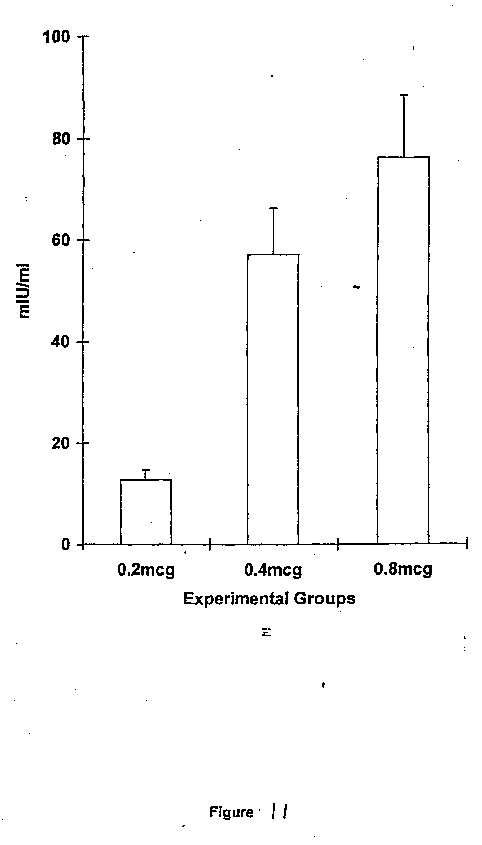

**Induction of anti HBV immune response:** Three groups of mice were followed for anti-HBs antibody titers post-inoculation with three different doses of the BioHepB vaccine. A significant difference between a dose of 0.2mcg compared with 0.4mcg, and 0.8mcg was observed. Vaccination with 0.2mcg induced anti-HBs titers of $12.65 \pm 7.28$ mlU/ml compared with $57.05 \pm 35.85$ and $76.20 \pm 82.31$ mlU/ml, following inoculation with 0.4 and 0.8 mcg respectively ($p<0.05$ and $p<0.03$ for 0.4 and 0.8 vs. 0.2 respectively, and $p = 0.2$ for the difference between 0.4 and 0.8 mcg; FIGURE 11). Thereby a vaccination dose of 0.4 mcg, administered i.p., was used in the study.

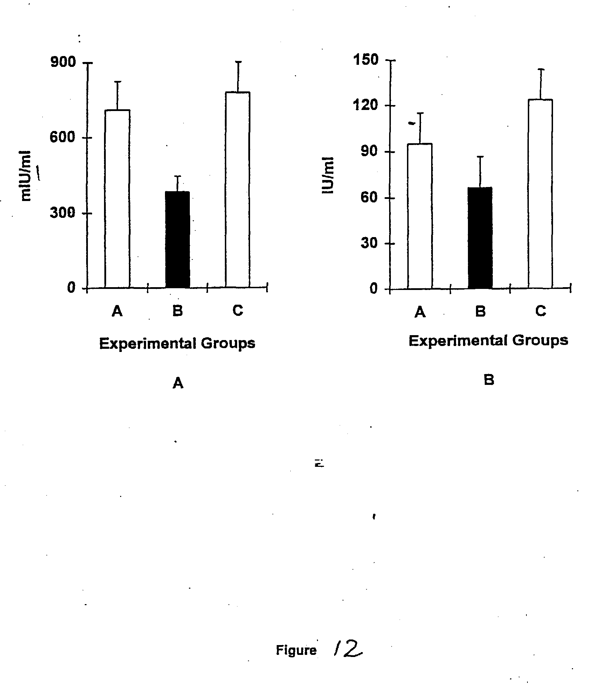

**Evaluation of the effects of tolerance induction on anti-viral humoral immune response:** Induction of anti-HBV peripheral immune tolerance was evaluated by measuring anti-HBsAg antibody production at monthly intervals in mice fed prior to HBV vaccination. Administration of HBV proteins prior to BioHepB.vaccination markedly downregulated the anti-viral humoral immune response. Thirty days following the first inoculation of the vaccine, serum anti-HBs antibody levels were $385 \pm 154$ vs. $709 \pm 390$ and $777 \pm 346$ mlU/ml) in tolerized, HBV-envelope proteins fed (group B), compared with non-tolerized non-fed controls (group A), and BSA-fed (group C), controls respectively ($p<0.013$, FIGURE 12A). Moreover, thirty days following the second inoculation with the BioHepB vaccine, anti-HBs antibody titers were $66495 \pm 44007$ mlU/ml vs. $95257 \pm 78320$ and $123607 \pm 76130$ mlU/ml, in tolerized vs. non-tolerized controls (groups A and C, $p<0.05$, FIGURE 12B).

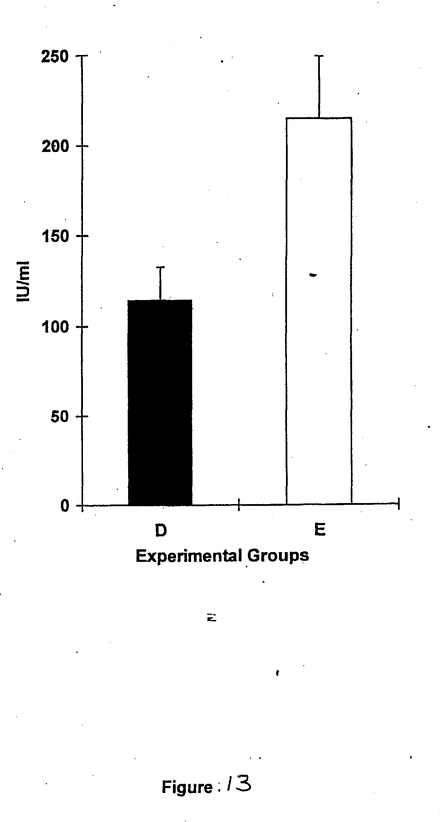

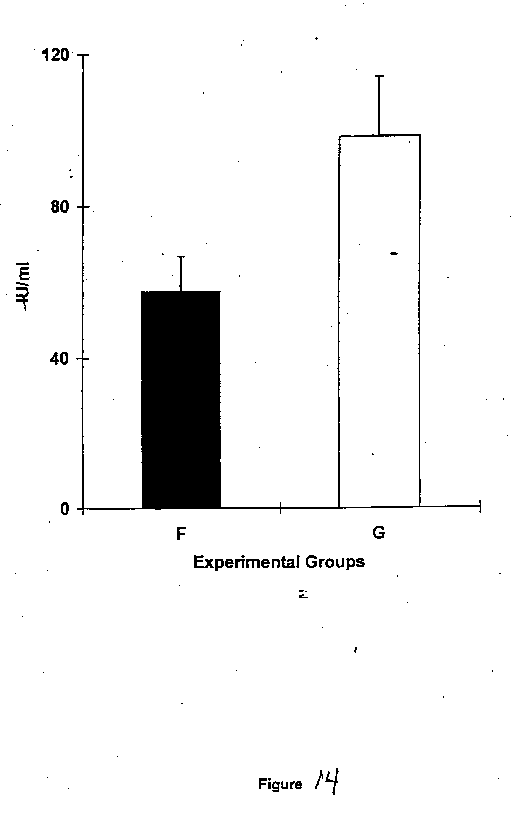

**Evaluation of the effect of oral tolerance induction on pre-existing anti-HBV immune response**: The effect of tolerance induction towards HBV proteins on pre-existing anti-viral immune response was evaluated by measuring anti-HBs antibody titers in anti-HBV immunized mice orally administered with BioHepB antigens or BSA. The feeding of HBV antigens immediately after exposure to HBV vaccination, reduced anti-HBs titers to $114024 \pm 94097$ mlU/ml in tolerized (group D) compared with $214776 \pm 148897$ mlU/ml in non-tolerized (group E) controls, 60 days following BioHepB inoculation ($p = 0.15$, FIGURE 13). Moreover, feeding of HBV antigens thirty days following two immunizations with the vaccine, significantly reduced anti-HBs antibody titers to $57354 \pm 37673$ vs. $98195 \pm 57256$ mlU/ml, in tolerized, HBV-envelope proteins fed, vs. non-tolerized, BSA-fed, controls as measured on day 60, thirty days following the second immunization (groups F and G respectively, $P<0.01$, FIGURE 14). Oral administration of HBV-envelope proteins without immunization had no effect on anti-HBs antibody titers and their anti-HBs serum levels were undetectable (group H, not shown).

**Summary**

This example demonstrates that oral administration of envelope proteins can induce tolerance towards HBV-epitopes. Feeding of PreS1 + preS2 + HBsAg recombinant complex of the HBV envelope proteins into naïve animals downregulated the anti-HBV immune response upon vaccination (groups B and C). In addition this example demonstrates that oral administration of HBV proteins to previously immunized mice induced tolerance towards HBV-envelope peptides (experimental groups D and E). Moreover, tolerance induction markedly downregulated the secondary anti-viral humoral immune response. Induction of anti-HBV immune tolerance, following two inoculations of the vaccine, effectively inhibited anti-HBS antibody production (experimental groups F and G).

**Example 7 HBV infection of *Tupaia***

The present example uses a small animal model, *Tupaia belangeri*, that is the subject of a co-pending patent application (Brown et al., U.S. Patent Application Serial No. 08/876,635, filed on June 16,1997). This example is directed towards a demonstration of disease symptoms induced in *Tupaia belangeri* after infection by HBV that can be used to measure the effectiveness of therapeutic regimes.

**Materials and Methods**

**Subjects.** *Tupaia belangeri* were obtained from Duke University Vivarium, Durham NC, and housed at Albert Einstein College of Medicine, Animal Institute. *Tupaia* were housed at 70 degrees in 12 hour light/dark cycles, one or a mating pair of two shrews per cat cage or squirrel monkey cage, and given a diet of fresh fruit (grapes, banana, apple, and orange), dried cat food, and water. Each cage also contained a small nest box.

**HBV infection of *Tupaia*.** Tupaia were anesthetized using ketamine:xylazine at 94:1 (Ketaset at 100mg/ml from Fort Dodge Animal Health, Fort Dodge, Iowa and Rompum at 20 mg/ml, from Bayer, Shawnee Mission, Kansas) administered by intramuscular injection into the thigh, at a dose of 0.001ml/gm body weight. HBV inoculations were derived from a pool of ten HBV serpositive clinical specimens. For infection, the tail of a *Tupaia* was shaved and disinfected followed by intravenous injection of 0.1 ml of the HBV pool into the tail vein using a 27 gauge butterfly needle.

**Serology**. After shaving and disinfecting the underside of the tail, blood samples of 0.5 to 1.0 ml were collected using 27 gauge butterfly needles, from the tail vein of anesthetized *Tupaia*. Blood samples from *Tupaia* were collected before HBV innoculation and weekly thereafter. All HBV serological tests were performed at ENZO Clinical Laboratories (Farmingdale, NY) by the same procedures that are used for clinical specimens except for a series of samples from longterm infections where a polyclonal HBsAg test was used (Austral Biologicals, San Diego, CA). Serial dilutions of plasma in PBS were made to titer the *Tupaia* antibody levels to the informative range of the test. In one case where along term ALT (alanine aminotransferase) levels were measured by a commercial kit purchased from Sigma (St. Louis, MO) using the manufacturers directions.

**Histology.** Liver percutaneous biopsies were performed under general anaesthesia using sterile techniqes. A small vertical midline incision in the abdomen was used to expose the liver, a small wedge liver biopsy was removed after tying off a portion of the liver with a purse line suture using a taper needle and silk. Biopsy tissues were split: one portion was preserved in neutral formalin, and the other was frozen at -80°C. Formalin-fixed tissue was parafin embedded, sectioned, and stained with hematoxylin/eosin (H&E stain) for histological examination, trichrome stain for collagen, or reticulin stain, which stains *Tupaia* collagen.

*In situ* **PCR for HBV RNA**. Reactions were carried out according to the protocol described in Liu et al., (1997; J. Vir. 71:4079) except that HBV specific sequences for primers and probe were used. The sequences for these are as follows:

HBV A: 5'-TGCCTGAGTGC(T/A)GTATG-3'
HBV B: 5'-TAGGAGGCTGTAGGCAT-3'
HB Probe: 5'-TTTATAAGGGTCGATGTCCAT-3'

**Results**

**HBV Infection of *Tupaia*.** Evidence for the HBV infection of *Tupaia* innoculated with HBV carrier sera is shown in Table 3 below.

Table 3

| HBV antigens in Tupaia: serological evidence of infection | | | | | | |
|---|---|---|---|---|---|---|
| Antigen/Animals | Preinfection | 5 min | 2 hr. | 24 hr. | 4 days | 10 days |
| A.S-Ag | | | | | | |
| 1 | - | - | + | + | + | - |
| 2 | - | - | nt | + | + | - |
| 3 | - | nt | nt | nt | + | - |
| 4 | - | nt | nt | nt | + | - |
| B.E-Ag | Preinfection | 6 days | 9 days | 17 days | 24 days | 60 days |
| 5 | - | - | + | + | + | - |
| 6 | - | - | - | + | + | - |
| 7 | - | - | - | - | + | - |

In HBV infected Tupaia, HBV surface antigen (HBsAg) was detected beginning at 2 hours after innoculation, peaked at 24 to 48 hours, and persisted for 4 to 6 days in all treated *Tupaia*, Table 3a. HBsAg production from longer timepoints were negative with the monoclonal assay. However, when a polyclonal antibody kit was substituted, HbsAg continued to be detected throughout the course of the time of the study (10 months). E-antigen, also known as HBeAg, is the serum soluble modification of viral core protein and, in man, the marker for active viral replication. HBeAg was detected in blood samples from all three *Tupaia* tested after HBV innoculation, Table 3b. Detection of HBeAg secretion extended from 9 to 24 days in consecutive blood samples. In control untreated *Tupaia*, no HBV viral antigens were detected in the blood. Similarly, in pretreatment blood samples from HBV innoculated tupaia, no HBV was detected, indicating that the *Tupaia* used had no previous exposure to human HBV. From this serological data we conclude that the Tupaia innoculated with HBV carrier sera were infected, and that they replicate virus.

**Immunological response of *Tupaia* to HBV antigens**. Since the host immune response to HBV is an important component in the necroinflammatory reaction in infected human liver, the ability for HBV antigens to invoke an immune response in *Tupaia* was tested. Plasma antibody levels to HBV viral proteins were measured after infection of *Tupaia* as a measure of the humoral immune response to HBV. Antibodies directed against HBsAg were not detected in untreated *Tupaia*, nor in blood samples taken from the *Tupaia* prior to their inoculation with HBV. As shown in Table 4 below, detection of antibodies took place at different times for each of the subjects.

TABLE 4

| Antibody levels after HBV inoculation | | | |
|---|---|---|---|
| week | Tupaia-1 | Tupaia-2 | Tupaia-3 |
| 0 | 10 | 10 | 10 |
| 4 | 10 | 10 | 4550 |
| 7 | 10,000 | 110 | 7000 |
| 12 | 3,050 | 24,500 | 220,000 |

One animal produced detectable levels after only 4 weeks. A second subject produced detectable levels beginning 7 weeks after infection. All three subjects were inoculated a second time with HBV. When tested all three subjects were positive for antibodies with titers that varied between $2 \times 10^3$ to $2 \times 10^5$ for individual HBV infected *Tupaia* after the booster response. These results show that after HBV infection, *Tupaia* are able to produce a humoral immune response to the virus. The variablity in timing and magnitude of antibody production is similar to that seen among human patients.

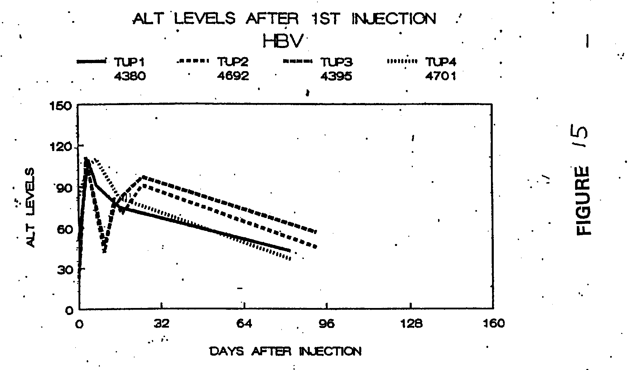

**Hepatocyte death in HBV infected *Tupaia*.** In HBV-infected human liver tissue, the host cellular immune response to virally infected hepatocytes is thought to cause hepatocyte death. One marker of hepatocyte death is the release of amino transferases from lysed hepatocytes into the plasma. Alanine amino transferase (ALT) levels in plasma are a standard measure of hepatocyte death and liver injury in viral hepatitis. ALT levels in *Tupaia* plasma were determined before and after inoculation of *Tupaia* with HBV. Normal, untreated *Tupaia* ALT levels ranged from 13 to 40 units/ml, and average 25 units/ml, similar to normal human ALT levels. However, following HBV innoculation, ALT levels in *Tupaia* were elevated two to five fold over normal levels as shown in FIGURE 15. This rise can be considered to be an indication that as seen in humans, there have been cellular immune responses to HBV that have led to hepatocyte death. The duration of ALT elevation varied between individual *Tupaia*, in the most extreme case persisting for 10 months. Persistant or periodic ALT elevation over 6 months or longer time period is a characteristic of chronic hepatitis in humans where there is a continuous cycle of hepatocytes being infected and destroyed.

**Histological evidence of hepatitis and HBV replication**

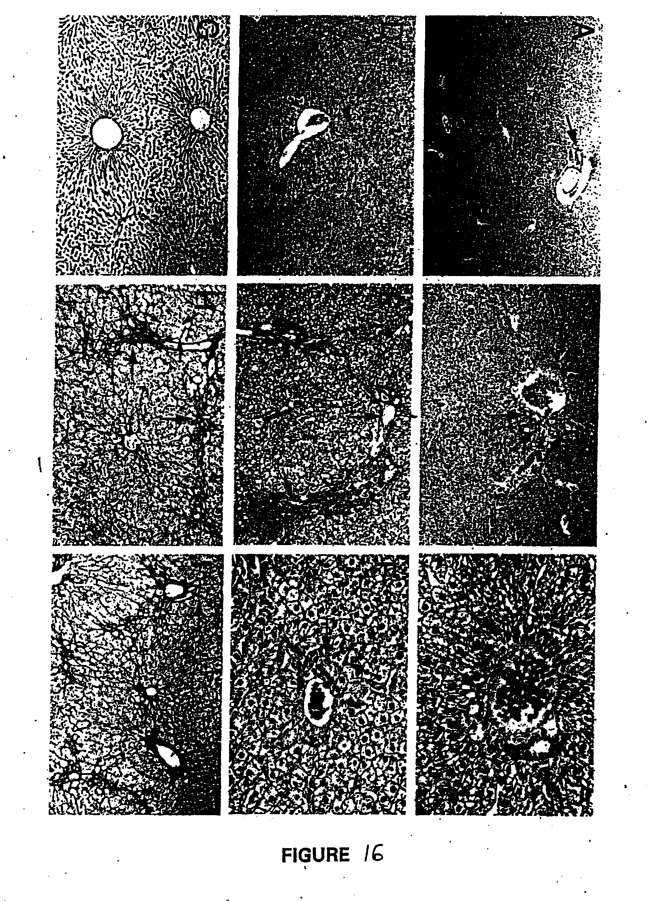

Exemplary photographs of biopsies from infected and uninfected *Tupaia* are shown in FIGURE 16.

Panel A: Untreated normal *Tupaia* liver section stained with hematoxylin ane eosin (50x) shows unremarkable morphology, with a single bile duct adjacent to the portal vein, and occasional lymphocytes. The liver structure is comparable to normal human liver.

Panel B: HBV-infected *Tupaia* liver section from a biopsy taken 2 months after HBV infection. H/E stain shows bile duct proliferation (50x). The field shown is representative of many fields viewed on this and adjacent sections of liver tissue.

Panel C: HBV-infected *Tupaia* liver section from a biopsy taken 2 months after HBV infection and stained with H/E (250x) shows altered hepatocyte morphology. Some cells are enlarged in size (balooning). Portal inflammation and mononuclear cells around the portal vein are seen.

Panel D: HBV-infected *Tupaia*, 10 month biopsy, liver section stained with H/E (50x) shows periportal and lobular lymphocyte infiltration.

Panel E: HBV-infected *Tupaia*, 6 months biopsy, liver section stained with trichrome (50x). Liver architecture is distorted by strands of collagen in this field. Strands of blue staining collagen are seen which connect at least three portal zones. Full formed cirrhotic nodules are not seen.

Panel F: HBV-infected *Tupaia*, same animal as in Panel D, 10 month liver biopsy stained with H/E (250x). Lymphocytes extend from the portal triad and surround degenerating hepatocytes in a piecemeal necrosis pattern.

Panel G: Uninfected *Tupaia* liver section stained with reticulin shows normal architecture, a network comparable to normal human liver.

Panel H: HBV-infected *Tupaia*, same animal as in Panel E, 6 months after HBV infection, stained for reticulin, shows groups of hepatocytes not individually surrounded by reticulin indicating regeneration, and thick strands of extracellual matrix indicating collagen deposition and hepatocellular collapse.

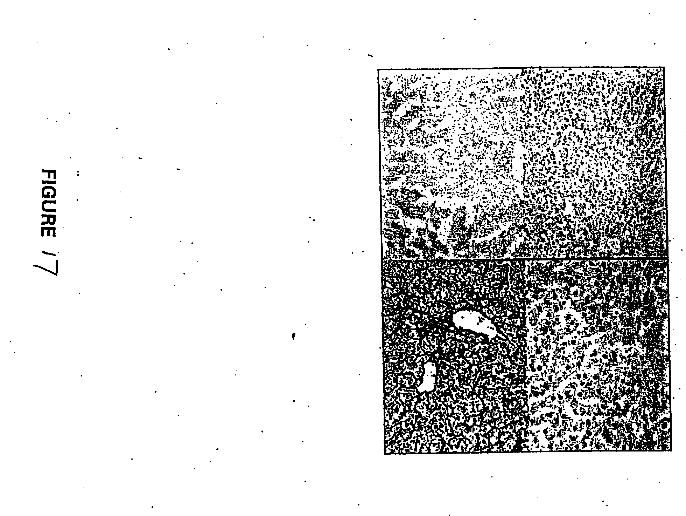

*In situ* PCR detection of HBV RNA is shown in FIGURE 17. The upper left panel shows the distribution of Hepatitis B RNA in the liver of a *Tupaia*, 6 months after infection by HBV. At higher magnification, (upper right panel) one can see infected hepatocytes and occasional Kuppfer cells. Viral RNA was not detected in an uninfected control animal, as seen in the lower left panel of FIGURE 17. The corresponding reticulin stain of the virally infected liver shows a diffuse increase in reticulin fibers in the region of the portal tract extending to the region of the central vein (lower right panel of FIGURE 17).

Summaries of three *Tupaia* cases are presented in more detail below; one control *Tupaia* that was uninfected and the two *Tupaia* that were examined at 6 months and 10 months after inoculation with HBV. Liver sections from each specimen were stained and then analyzed independently by two clinical pathologists.

Tupaia #1 Untreated adult *Tupaia*. The H&E stain demonstrates normal hepatic architecture. There is no evidence of inflammation in the portal tracts and there are rare mononuclear cells in the sinusoidal spaces. Hepatocytes and bile ductules are unremarkable. The reticulin stain, which stains for collagen deposition, does not show any increased reticulin. Hence, this liver tissue is within normal limits. No evidence was seen for the presence of HBV RNA after an *in situ* PCR reaction.

Tupaia # 2. Specimens obtained 6 months after inoculation with HBV. The H&E stain demonstrates normal hepatic architecture. There is widespread evidence of moderate, chronic inflammation in the portal tracts (portal hepatitis). The majority of these inflammatory cells are lymphocytes and plasma cells. Occasionally, the inflammation goes past the limiting plate (lobular hepatitis), although hepatocyte necrosis/ piecemeal necrosis is not evident. There are scattered mononuclear cells in the sinusoidal spaces. The hepatocytes show a diffuse and severe steatosis (fatty change), which is a common finding in HCV-infected human liver. Serological tests gave negative results for HCV, indicating that in this Tupaia the steatosis did not result from contaminating or previous HCV infection. Rare ground glass cells are evident, which is a common finding in HBV infected human liver and results from HBsAg accumulation within hepatocytes. The reticulin stain for collagen deposition shows a diffuse and moderate increase in reticulin fibers centered at the portal tracts and rarely extending to the region of the central vein. These histologic findings are consistent with moderately severe, chronic viral hepatitis. In results of tests for HBV replication in adjacent tissue sections, viral RNA was detected in occasional hepatocytes using the RT in situ PCR technique. The viral nucleic acid localized to the region of the nuclear membrane. This demonstrates active HBV replication in the liver at the time of biopsy.

Tupaia # 3. Specimens obtained 10 months after inoculation with HBV. The H&E stain demonstrates normal hepatic architecture. There is minimal evidence of chronic inflammation in the portal tracts (portal hepatitis). There are rare mononuclear cells in the sinusoidal spaces and the hepatocytes are unremarkable. The reticulin stain for collagen shows a focal and mild increase in reticulin fibers centered at the portal tracts, indicating deposition of fine strands of connective tissue (periportal fibrosis). These histologic findings are consistent with mild, chronic hepatitis. Viral RNA was not detected at the time of biopsy (10 months post innoculation).

### Summary

A primitive lower primate, *Tupaia belangeri*, can be infected with HBV such that a number of characteristics analogous to human disease can be observed. These disease symptoms are useful markers for determining the effectiveness of a therapeutic process. Some of these markers, such as the presence of HBeAg, can be used to monitor the presence of an ongoing viral replication. Other markers, such as ALT levels and histological examinations for inflammatory responses can be used for the determination of the extent of secondary manifestations of infection.

### Example 8 Induction of oral tolerization to HBV antigens in *Tupaia*

This example is a demonstration that SIDR can be used to alleviate disease symptoms that are associated with viral infection. In this example, induction of oral tolerance is used to alleviate detrimental immune manifestations that have been described in the previous example after HBV infection of *Tupaia belangeri*. This example also serves as a further demonstration that induction of oral tolerance can be used to selectively down-regulate an antibody reaction to an antigen of a pathogen.

### Materials and Methods

**Subjects, Serology** and **Histology** were as described in the previous example

**Induction of Oral Tolerization.** Adult *tupaia* were fed 30 ng of HBsAg in a solution containing 1 mg fetal bovine serum carrier (10 doses, given every other day), before or after infection with HBV-infected human serum. HBsAg used as the tolerant was derived from the cell culture supernatant collected from human hepatocyte cell line IHBV6.7. The derivation and characterizationof this cell line has been described in co-pending patent application (Brown et al. U.S. Patent Application Serial No. 08/876/635, filed on June 16, 1997). At confluence, IHBV6.7 stationary cells produce 90ng/ml HBsAg. For no treatment controls, adult tupaia were fed 1 mg BSA without the HBsAg.

**Results**

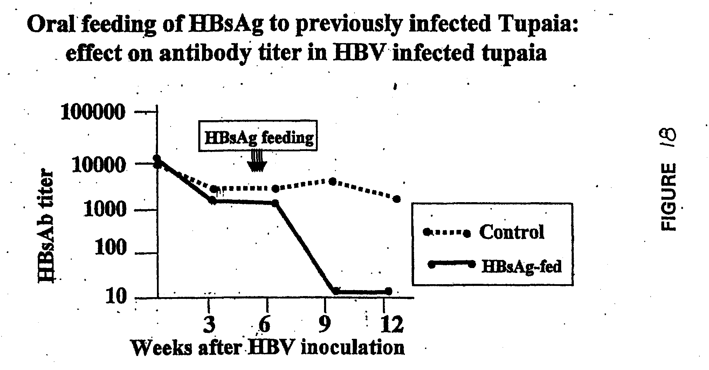

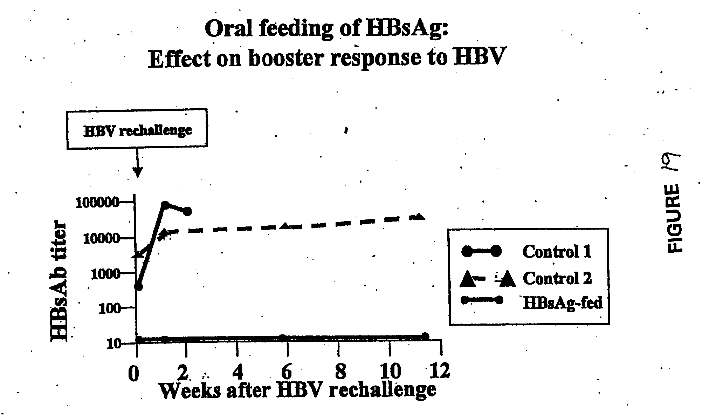

**Antibody Levels**. When a pair of Tupaia were infected with HBV, high levels of antibodies to surface antigen were detected (FIGURE 18). When one of the infected Tupaia was orally administered HBsAg 6 weeks post-infection, antibody levels were reduced by at least two orders of magnitude while the control continued to produce high levels of antibody. When these two animals were given a second inoculation the control subject showed a booster response as did a second control animal (FIGURE 19). However, the subject that had been orally tolerized as previously described failed to demonstrate any response to the HBV challenge. No detectable levels of HBV antibody were detected over a twelve week period.

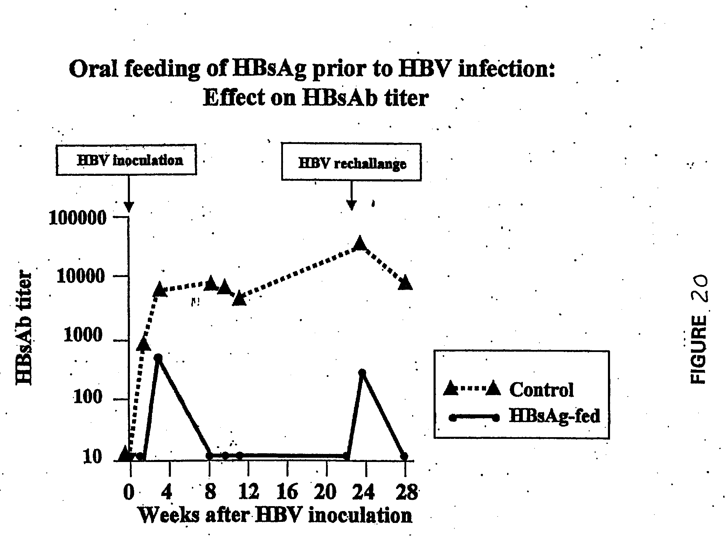

Tupaia were also tolerized prior to HBV infection. Results from this experiment are shown in FIGURE 20. The subject which was treated by oral administration of HBsAg showed an initial response that was 10 fold lower than the control. When tested at 8 weeks post-infection, antibody levels in the tolerized animal were undetectable and remained so until a rechallenge was administered at 21 weeks post infection. After rechallenge, antibody level response was 400 fold lower in the tolerized animal compared to the control. It also soon returned to undetectable levels.

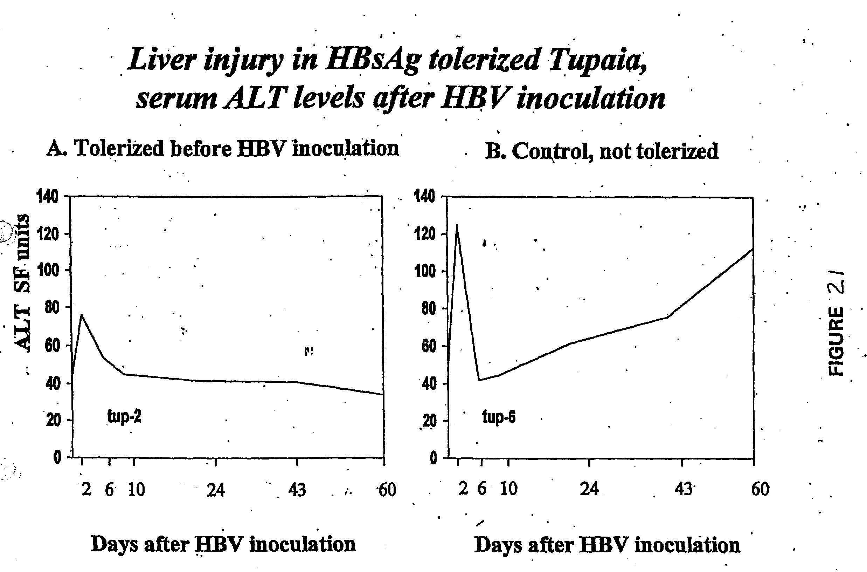

**ALT levels**. The subjects shown in FIGURE 20 were also tested for liver injury by measuring serum ALT levels. Results of these assays are shown in FIGURE 21. For both the tolerized and the control Tupaia, initial ALT peaks seen at two days pos-inoculation return to normal levels within ten days. In the control animal, the ALT starts to rise again from day 24 to 60 days post-inoculation which is diagnostic of continuing liver damage. In contrast, the tolerized subject maintained normal levels of ALT throughout the study.

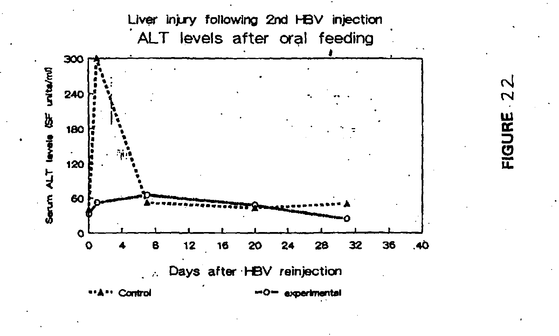

The subjects shown in FIGURE 19 who had booster HBV injections were also tested for liver injury by measuring serum ALT levels. Results of these assays are shown in FIGURE 22. The control HBV infected Tupaia shows a high ALT response to the second inoculation whereas the subject treated with oral tolerization demonstrates alsmost no response.

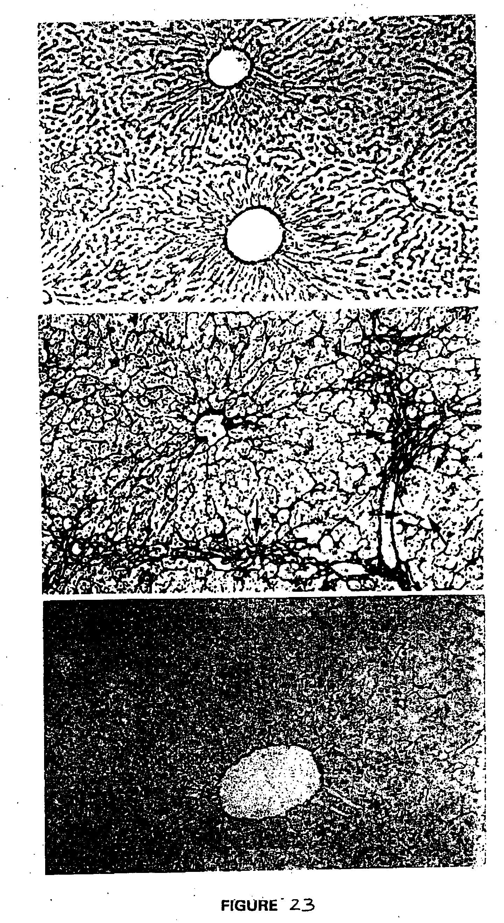

**Histology**. Liver biopsies from 3 HBV-infected, BSA-fed controls showed varying degrees of inflammatory end fibrotic lesions, (FIGURE 16 from the previous example). In contrast 2 HBsAg-fed infected *Tupaias* and 3 uninfected *Tupaias* were lacking evidence of inflamation or fibrosis. An example of this is shown in FIGURE 23 where the top panel shows normal liver specimen from an uninfected Tupaia. In contrast, the middle panel shows the unhealthy tissue seen in a specimen form a Tupaia infected with HBV. The arrows in the middle panel show the extensive fibrosis present 10 months after infection. The lower panel demonstrates the absence of any signs of a disease process 10 months after infection in a *Tupaia* which has undergone post-infection oral tolerance induction.

**Summary**

These further results demonstrate that the therapy of oral tolerization to HBV antigens ameliorated hepatic inflammation in HBV hepatitis in this animal model for liver pathology. Contrary to what may be expected, the abrogation of an immune response to an HBV surface antigen did not lead to a fulminant display of viremia. When the *tupaia* were tolerized to the surface antigen of HBV prior to infection, there was a dramatic decrease in the levels of antibody to the surface antigen. In addition, a therapeutic effect was noted in terms of a loss of inflammatory responses to the HBV infection that had been observed in the livers of the non-tolerized animals. This effect will allow a therapeutic regime in this and other pathogen systems where oral tolerization can be carried out with regard to selected antigens or epitopes that invoke a self-immune response while sustaining an immune response to other antigens or epitopes. This would allow a natural control of the infection by the subject.

Many obvious variations will be suggested to those of ordinary skill in the art in light of the above detailed description and examples of the present invention. All such variations are fully embraced by the scope and spirit of the invention as defined more particularly by the claims that follow.

**Claims**

1. Use of native or non-native antigen or antigens of a recipient or immunologically equivalent thereof, or native or to non-native antigen or antigens of a donor or both for the preparation of a pharmaceutical composition for use in a transplantation process comprising the step of establishing selective immune down regulation in said recipient, said recipient comprising immune cells, immune tissues, immune organs or components thereof from said donor.

2. The use of claim 1, wherein said immune cells, immune tissues, immune organs or components thereof are selected from the group consisting of T cells, dendritic cells, lymphocytes, peripheral blood cells, NK cells, haematopoietic stem cells, bone marrow and combinations thereof.

3. The use of claim 1, wherein said selective immune down regulation is established by orally tolerizing said recipient by administering said native or non-native antigen or antigens, said native or non-native antigen or antigens comprising one or more components selected or derived from the group consisting of (a) the cells, tissues or fragments thereof from the recipient, (b) the cells, tissues or fragments thereof from the donor, (c) the cells, tissues or fragments thereof complexed with antibodies from the recipient, (d) the cells, tissues or fragments thereof complexed with antibodies from the donor, or from partial digests of any of (a), (b), (c) and (d), any immunologically equivalent compound, and any combination of any of the foregoing.

4. The use of claim 1 whereby the transplantation process is to be used for preventing or treating graft-versus-host rejection in a recipient.

5. The use of claim 4, wherein said native or non-native antigen or antigens are derived from said recipient.

6. The use of claim 4, wherein said native or non-native antigen or antigens are derived from said recipient and comprises native or non-native antigen or antigens from said recipient and said donor.

7. The use of claim 4, wherein said native or non-native antigen or antigens are derived from the group consisting of T cells, dendritic cells, lymphocytes, peripheral blood cells, NK cells, haematopoietic stem cells, bone marrow and combinations thereof.

8. The use of claim 7, wherein said native or non-native antigen or antigens comprises peripheral blood cells.

9. Use of any or all antigens used as a vaccine, immunization agent, adjuvant or immunologically equivalent compound, and any combination of any of the foregoing for the preparation of a pharmaceutical composition for treating or preventing undesirable immunological consequences of vaccination or immunization in a subject comprising the step of establishing selective immune down regulation in said subject.

10. A composition of matter comprising trained or programmed cells, tissues or organ, which when introduced into a recipient exhibits or establishes selective immune down regulation in said recipient.

11. The composition of claim 10, wherein said trained or programmed cells, tissues or organ are derived from a subject that has been previously tolerized.

12. The composition of claim 10, wherein said trained or programmed cells, tissues or organ are derived from a subject allogenic to said recipient.

13. The composition of claim 10, wherein said trained or programmed cells, tissues or organ are derived from a subject xenogenic to said recipient.

14. The composition of claim 10, wherein said trained or programmed cells, tissues or organ have been tolerized *in vitro*, outside of said recipient, before being introduced into said subject.

15. The composition of claim 10, comprising cells, tissues, or organ, which when introduced into said recipient, exhibit or establish tolerization in said recipient to a specific antigen or antigens.

16. Use of native or non-native antigen or antigens comprising material derived from cells, tissues, organs, or components thereof afflicted or a normal target of Crohn's disease, said native or non-native antigen or antigens being derived from allogeneic sources, xenogeneic sources, autologous sources, and combinations of said sources, and immunologically equivalent compounds, and combinations of any of the foregoing for the preparation of a pharmaceutical composition for treating Crohn's disease in a subject comprising the steps of establishing selective immune down regulation in said subject to native or non-native antigens of the diseased cell, tissue or organ in said subject.

17. The use of claim 16, wherein said cells, tissues, organs, or components thereof afflicted or a normal target of Crohn's disease comprise intestinal cells.

18. Use of native or non-native antigen or antigens comprising material derived from cells, tissues, organs, or components thereof afflicted or a normal target of primary sclerosing cholangitis disease, said native or non-native antigen or antigens being derived from allogeneic sources, xenogeneic sources, autologous sources, and combinations of said sources, and immunologically equivalent compounds, and combinations of any of the foregoing for the preparation of a pharmaceutical composition for treating primary sclerosing cholangitis disease in a subject comprising the steps of establishing selective immune down regulation in said subject to native or non-native antigens of the diseased cell, tissue or organ in said subject.

19. Use of native or non-native antigen or antigens comprising material derived from cells, tissues, organs, or components thereof afflicted or a normal target of primary biliary cirrhosis disease, said native or non-native antigen or antigens being derived from allogeneic sources, xenogeneic sources, autologous sources, and combinations of said sources, and immunologically equivalent compounds and combinations of any of the foregoing for the preparation of a pharmaceutical composition for treating primary biliary cirrhosis disease in a subject comprising the steps of establishing selective immune down regulation in said subject to native or non-native antigens of the diseased cell, tissue or organ in said subject.

20. Use of native or non-native antigen or antigens comprising material derived from cells, tissues, organs, or components thereof afflicted or a normal target of Celliac's disease, said native or non-native antigen or antigens being derived from allogeneic sources, xenogeneic sources, autologous sources, and combinations of said sources, and immunologically equivalent compounds, and combinations of any of the foregoing for the preparation of a pharmaceutical composition for treating primary Celliac's disease in a subject comprising the steps of establishing selective immune down regulation in said subject to native or non-native antigens of the diseased cell, tissue or organ in said subject.

21. Use of native or non-native antigen or antigens comprising material derived from cells, tissues, organs, or components thereof afflicted or a normal target of primary autoimmune chronic active hepatitis disease, said native or non-native antigen or antigens being derived from allogeneic sources, xenogeneic sources, autologous sources, and combinations of said sources, and immunologically equivalent compounds, and combinations of any of the foregoing for the preparation of a pharmaceutical composition for treating primary autoimmune chronic active hepatitis disease in a subject comprising the steps of establishing selective immune down regulation in said subject to native or non-native antigens of the diseased cell, tissue or organ in said subject.

22. Use of native or non-native antigen or antigens comprising material derived from cells, tissues, organs, or components thereof afflicted or a normal target of chronic liver rejection disease, said native or non-native antigen or antigens being derived from allogeneic sources, xenogeneic sources, autologous sources, and combinations of said sources, and immunologically equivalent compounds, and combinations of any of the foregoing for the preparation of a pharmaceutical composition for treating chronic liver rejection disease in a subject comprising the steps of establishing selective immune down regulation in said subject to native or non-native antigens of the diseased cell, tissue or organ in said subject.

23. Use of native or non-native antigen or antigens comprising material derived from cells, tissues, organs, or components thereof afflicted or a normal target of immune-mediated liver fibrosis disease, said native or non-native antigen or antigens being derived from allogeneic sources, xenogeneic sources, autologous sources, and combinations of said sources, and immunologically equivalent compounds, and combinations of any of the foregoing for the preparation of a pharmaceutical composition for treating immune-mediated liver fibrosis disease in a subject comprising the steps of establishing selective immune down regulation in said subject to native or non-native antigens of the diseased cell, tissue or organ in said subject.

24. Use of native or non-native antigen or antigens comprising material derived from cells, tissues, organs, or components thereof afflicted or a normal target of immune-mediated vascular disorder, said native or non-native antigen or antigens being derived from allogeneic sources, xenogeneic sources, autologous sources, and combinations of said sources, and immunologically equivalent compounds, and combinations of any of the foregoing for the preparation of a pharmaceutical composition for treating immune-mediated vascular disorder in a subject comprising the steps of establishing selective immune down regulation in said subject to native or non-native antigens of the diseased cell, tissue or organ in said subject.

25. Use of native or non-native antigen or antigens comprising material derived from cells, tissues, organs, or components thereof afflicted or a normal target of immune-mediated muscle disorder, said native or non-native antigen or antigens being derived from allogeneic sources, xenogeneic sources, autologous sources, and combinations of said sources, and immunologically equivalent compounds, and combinations of any of the foregoing for the preparation of a pharmaceutical composition for treating immune-mediated muscle disorder in a subject comprising the steps of establishing selective immune down regulation in said subject to native or non-native antigens of the diseased cell, tissue or organ in said subject.

26. The use of claim 25, wherein said immune-mediated muscle disorder affects a muscle member selected from the group consisting of smooth muscle, striated muscle, blood vessel muscles, and combinations of any of the foregoing.

27. The composition of any one of claims 10 to 15 which is a pharmaceutical composition.

FIGURE 1

Figure 2

FIGURE 3

FIGURE 4

FIGURE 5

EP 1 072 271 A2

FIGURE 6

30

FIGURE 7

FIGURE 8

FIGURE 9

FIGURE 10

Figure 11

Figure /2

Figure : 13

Figure /4

FIGURE 15

FIGURE 16

FIGURE 17

Oral feeding of HBsAg to previously infected Tupaia:
effect on antibody titer in HBV infected tupaia

FIGURE 18

Oral feeding of HBsAg:
Effect on booster response to HBV

FIGURE 19

EP 1 072 271 A2

Oral feeding of HBsAg prior to HBV infection:
Effect on HBsAb titer

FIGURE 20

44

## Liver injury in HBsAg tolerized Tupaia, serum ALT levels after HBV inoculation

### A. Tolerized before HBV inoculation

### B. Control, not tolerized

Days after HBV inoculation

Days after HBV inoculation

FIGURE 2.1

45

Liver injury following 2nd HBV injection
ALT levels after oral feeding

FIGURE 22

FIGURE 23